# EUROPEAN PATENT SPECIFICATION

(11) **EP 4 028 052 B1**
(45) Date of publication and mention of the grant of the patent: **15.07.2026**
(21) Application number: 19848910.6
(22) Date of filing: 04.11.2019
(51) Int. Cl.: A61K 39/395, A61P 35/00, A61N 1/36

(54) **A METHOD OF TREATING CANCER BY APPLYING ALTERNATING ELECTRIC FIELDS AND ADMINISTERING CHECKPOINT INHIBITORS**
VERFAHREN ZUR BEHANDLUNG VON KREBS DURCH ANLEGEN WECHSELNDER ELEKTRISCHER FELDER UND VERABREICHUNG VON CHECKPOINT-INHIBITOREN
PROCÉDÉ POUR LE TRAITEMENT DU CANCER PAR L'APPLICATION DE CHAMPS ÉLECTRIQUES ALTERNATIFS ET L'ADMINISTRATION D'INHIBITEURS DE POINTS DE CONTRÔLE

(30) Priority: 10.09.2019 US 201962898290 P
(43) Date of publication of application: 20.07.2022
(73) Proprietor: Novocure GmbH, 6340 Baar (CH)
(72) Inventor: CHEN, Dongjiang, Gainesville, Florida 32608 (US); TRAN, David, Gainesville, Florida 32606 (US)
(74) Representative: Carpmaels & Ransford LLP
(86) International application number: PCT/US2019/059650
(87) International publication number: WO 2021/050093

(56) References cited:
- WO-A1-2019/100016
- US-A1- 2018 008 708
- DATABASE EMBASE [online] ELSEVIER SCIENCE PUBLISHERS, AMSTERDAM, NL; 1 August 2019 (2019-08-01), DIAMANT G ET AL: "Evaluating the compatibility of tumor treating electric fields with key antitumoral immune functions", Database accession no. EMB-631167799
- DIAMANT G ET AL: "Evaluating the compatibility of tumor treating electric fields with key antitumoral immune functions", NEURO-ONCOLOGY 20190801 OXFORD UNIVERSITY PRESS NLD, vol. 21, no. Supplement 3, 1 August 2019 (2019-08-01), pages iii60 CONF 20190919 to 20190922 Lyon - 14th Meet, ISSN: 1523-5866
- DATABASE EMBASE [online] ELSEVIER SCIENCE PUBLISHERS, AMSTERDAM, NL; 1 August 2019 (2019-08-01), DIAMANT G ET AL: "Evaluating the compatibility of tumor treating electric fields with key antitumoral immune functions", XP002798817, Database accession no. EMB-631167799
- NEURO-ONCOLOGY 20190801 OXFORD UNIVERSITY PRESS NLD, vol. 21, no. Supplement 3, 1 August 2019 (2019-08-01), pages iii60 CONF 20190919 to 20190922 Lyon - 14th Meet, ISSN: 1523-5866
- VOLOSHIN T ET AL: "Immunomodulatory Effect of Tumor Treating Fields (TTFields) Results in Enhanced Antitumor Efficacy When Combined with Anti-PD-1 Therapy in Mouse Model of Lung Cancer", INTERNATIONAL JOURNAL OF RADIATION: ONCOLOGY BIOLOGY PHYSICS, vol. 104, no. 1, 1 May 2019 (2019-05-01), pages 237, XP085656556, ISSN: 0360-3016, DOI: 10.1016/J.IJROBP.2019.01.027
- VOLOSHIN T ET AL: "Immunogenic Cell Death Induced by Tumor Treating Fields (TTFields) Enhances Efficacy When Combined with Anti-PD-1 Therapy in Lung and Colon Cancer Animal Models", INTERNATIONAL JOURNAL OF RADIATION: ONCOLOGY BIOLOGY PHYSICS, PERGAMON PRESS, USA, vol. 105, no. 1, 1 September 2019 (2019-09-01), XP085823065, ISSN: 0360-3016, [retrieved on 20190914], DOI: 10.1016/J.IJROBP.2019.06.1031
- ONCOLOGYPRO: "Immunomodulatory Effects of Tumor Treating Fields (TTFields) on Lung Cancer Models | OncologyPRO", 5 April 2019 (2019-04-05), XP055687644, Retrieved from the Internet <URL:https://oncologypro.esmo.org/meeting-resources/european-lung-cancer-congress-2019/Immunomodulatory-Effects-of-Tumor-Treating-Fields-TTFields-on-Lung-Cancer-Models> [retrieved on 20200421]
- CHEN D ET AL: "Ttfields Induces Immunogenic Cell Death and Sting Pathway Activation Through Cytoplasmic Double-Stranded DNA in Glioblastoma Cells", INTERNATIONAL JOURNAL OF RADIATION: ONCOLOGY BIOLOGY PHYSICS, PERGAMON PRESS, USA, vol. 105, no. 1, 1 September 2019 (2019-09-01), XP085823145, ISSN: 0360-3016, [retrieved on 20190914], DOI: 10.1016/J.IJROBP.2019.06.1115
- TAKAYUKI OHKURI ET AL: "STING contributes to anti-glioma immunity via triggering type-I IFN signals in the tumor microenvironment", JOURNAL FOR IMMUNOTHERAPY OF CANCER, BIOMED CENTRAL, LONDON, UK, vol. 2, no. Suppl 3, 6 November 2014 (2014-11-06), pages P228, XP021202496, ISSN: 2051-1426, DOI: 10.1186/2051-1426-2-S3-P228
- DESAI KUNAL ET AL: "The Role of Checkpoint Inhibitors in Glioblastoma", TARGETED ONCOLOGY, SPRINGER PARIS, PARIS, vol. 14, no. 4, 9 July 2019 (2019-07-09), pages 375 - 394, XP036898289, ISSN: 1776-2596, [retrieved on 20190709], DOI: 10.1007/S11523-019-00655-3

## Description

### TECHNICAL FIELD

This application relates to methods for reducing viability of cancer cells by activation of the sting pathway with TTFields, and the invention provides a checkpoint inhibitor for use in a method of treating cancer in a subject.

### BACKGROUND

G. Diamant et al., in , 'Evaluating the compatibility of tumor treating electric fields with key antitumoral immune functions' August 2019, assert that preclinical data showed that all antitumoral T cell functions examined, but proliferation, were unhindered by TTFields, and that clinical data showed that TTFields may shift treated tumors to a state more conducive of antitumoral immune responses. Chen et al., in 'Ttfields Induces Immunogenic Cell Death and Sting Pathway Activation Through Cytoplasmic Double-Stranded DNA in Glioblastoma Cells' September 2019, conclude that their results provide compelling evidence that TTFields activates the innate immune system in GBM cells, Kirson et al., in 'Disruption of Cancer Cell Replication by Alternating Electric Fields' May 2004, conclude that their findings demonstrate the potential applicability of the described electric fields as a novel therapeutic modality für malignant tumors.

Tumor Treating Fields (TTFields) are an effective anti-neoplastic treatment modality delivered via non-invasive application of low intensity, intermediate frequency (e.g., 100-500 kHz), alternating electric fields. TTFields exert directional forces on polar microtubules and interfere with the normal assembly of the mitotic spindle. Such interference with microtubule dynamics results in abnormal spindle formation and subsequent mitotic arrest or delay. Cells can die while in mitotic arrest or progress to cell division leading to the formation of either normal or abnormal aneuploid progeny. The formation of tetraploid cells can occur either due to mitotic exit through slippage or can occur during improper cell division. Abnormal daughter cells can die in the subsequent interphase, can undergo a permanent arrest, or can proliferate through additional mitosis where they will be subjected to further TTFields assault. Giladi M et al. Sci Rep. 2015;5:18046.

In the in vivo context, TTFields therapy can be delivered using a wearable and portable device (Optune^{®}). The delivery system includes an electric field generator, 4 adhesive patches (non-invasive, insulated transducer arrays), rechargeable batteries and a carrying case. The transducer arrays are applied to the skin and are connected to the device and battery. The therapy is designed to be worn for as many hours as possible throughout the day and night.

In the preclinical setting, TTFields can be applied in vitro using, for example, the Inovitro^{™} TTFields lab bench system. Inovitro^{™} includes a TTFields generator and base plate containing 8 ceramic dishes per plate. Cells are plated on a cover slips placed inside each dish. TTFields are applied using two perpendicular pairs of transducer arrays insulated by a high dielectric constant ceramic in each dish. The orientation of the TTFields in each dish is switched 90° every 1 second, thus covering different orientation axes of cell divisions.

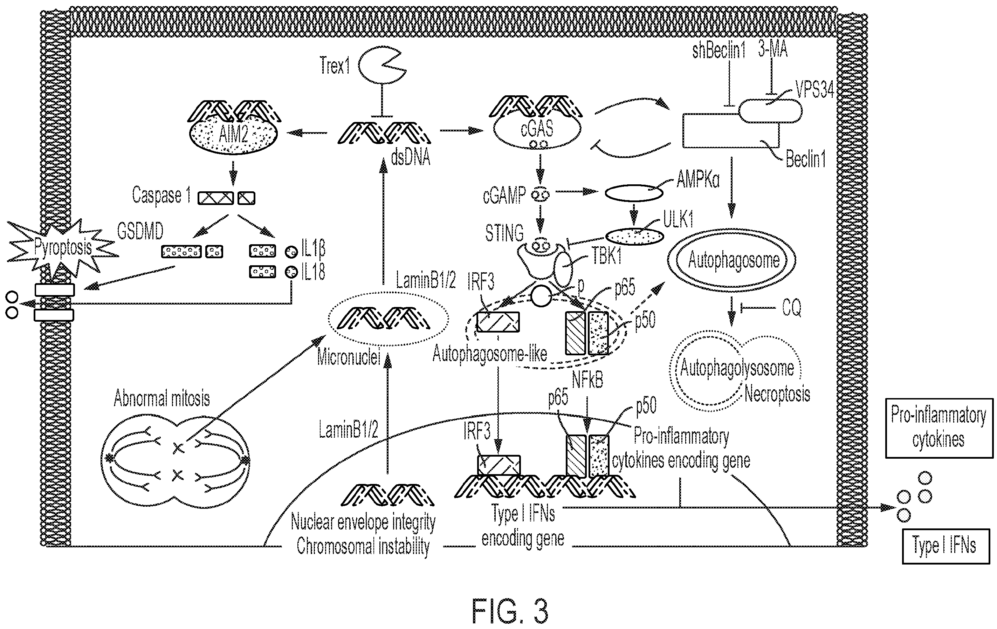

Recently the immune sensing molecule cyclic GMP-AMP synthase (cGAS)-Stimulator of Interferon Genes (STING, encoded by *TMEM 173*) pathway was identified as an important component of cytosolic DNA sensing and plays an important role in mediating the immune response in cells. Ghaffari et al., British Journal of Cancer, volume 119, pages 440-449 (2018); *see*, *e.g*., Figure 3. Activation of the STING pathway mediates the immune response by responding to abnormalities in the cells (e.g., the presence of cytoplasmic double-stranded DNA (dsDNA)).

Checkpoint proteins function as inhibitors of the immune system (e.g., T-cell proliferation and IL-2 production) which can lead. Azoury et al., Curr Cancer Drug Targets. 2015;15(6):452-62. Checkpoint proteins can have a deleterious effect with respect to cancer by shutting down the immune response. Blocking the function of checkpoint proteins can be used to activate dormant T-cells to attack cancer cells. Checkpoint inhibitors are cancer drugs that inhibit checkpoint proteins in order to recruit the immune system to attack cancer cells.

Thus, there is an interest in using checkpoint inhibitors as a cancer treatment to block the activity of checkpoint proteins enabling the production of cytokines and recruitment of T-cells to attack cancerous cells and are an active area in immunotherapy drug development.

What is needed are methods for activating the immune response and enhance and stimulate the response to cancer treatments, such as checkpoint inhibitors.

### SUMMARY

The present invention provides a checkpoint inhibitor for use in a method of treating cancer, in accordance with the appended claims. Also disclosed herein are various methods. Disclosed methods for treatment of the human or animal body by surgery or therapy and diagnostic methods practised on the human or animal body are not claimed by the present invention, but are useful for understanding aspects of the claimed invention. Any references in the description to methods of treatment of the human or animal body by surgery or therapy and diagnostic methods practised on the human or animal body refer to the compounds, pharmaceutical compositions and medicaments of the present invention for use in such methods.

As described herein, exposing cancer cells (e.g., glioblastoma cells) to TTFields induces the STING pathway leading to production of pro-inflammatory cytokines (e.g., Type I interferons) and pyroptosis. In one aspect, activating the STING pathway with TTFields is analogous to "vaccinating" the cancer cell, making the cancer cell especially susceptible to treatment with anti-cancer drugs such as checkpoint inhibitors. Thus, exposing cancer cells to TTFields continuously, discontinuously, or intermittently can make cancer cells susceptible to further treatment by inducing the STING pathway followed by treatment with one or more checkpoint inhibitors and/or other oncology drugs.

### BRIEF DESCRIPTION OF THE FIGURES

Figure 1 shows that TTFields can induce the formation of cytoplasmic micronuclei (double stranded DNA or dsDNA) in glioblastoma (GBM) cells exposed to TTFields (TTFields) vs control GBM cells (Control);
Figure 2 shows that nuclear lamin B1 structures are disrupted after exposure to TTFields, leading to release of dsDNA into the cytoplasm in LN827 cells;
Figure 3 shows an example of the biochemical pathways induced by cytoplasmic dsDNA (proinflammatory (STING) and pyroptosis pathways);
Figure 4 shows that cGAS and AIM2 independently co-localize with micronuclei in response to exposure to TTFields;
Figure 5 provides charts of percentage of AIM2/cGAS co-localization with micronuclei from the results of Figure 4;
Figure 6 shows the phosphorylation of IRF3 and p65 after exposure to TTFields for one day in U87 and LN827 cells;
Figure 7 shows that TTFields induce Type I IFN response and pro-inflammatory cytokines downstream of STING;
Figure 8 shows that STING is degraded after becoming activated by TTFields in GBM cells (LN428 human cells and KR158 mouse cells);
Figure 9 shows that STING is required for inflammatory responses induced by dsDNA and TTFields treatment in GBM cells (LN428 human cells, KR158 mouse cells, and F98 rat cells);
Figure 10 shows that autophagy and dsDNA or TTFields synergistically induce STING-dependent proinflammatory responses in KR158 and F98 GBM cells;
Figure 11 shows that TTFields-induced inflammatory cytokine production is dependent on STING and AIM2 in the F98 Rat Glioma Model;
Figure 12 shows that tumor size is corelated with fold changes in inflammatory cytokine expression in response to TTFields;
Figure 13 provides exemplary heat maps show that recruitment of CD45 cells into GBM is lower in GBM lacking STING and AIM2 in the F98 Rat glioma model;
Figure 14 provides exemplary heat maps show that CD3 (T cells) recruitment is lower in GBM lacking STING and AIM2;
Figure 15 provides exemplary heat maps showing that in GBM lacking STING and AIM2, DC/Macrophage recruitment is lower and MDSC recruitment is higher;
Figure 16 provides quantitative results of the data in Figure 17;
Figure 17 shows 'ghosting' induced by three days of exposure to TTFields in human GBM cell lines LN308 and LN827;
Figure 18 shows that TTFields induce membrane damage and decrease GSDMD in U87 GBM cells exposed to TTFields;
Figure 19 shows that TTFields induce membrane damage and cleaves GSDMD in human leukemia monocyte cell line THP-1 macrophages;
Figure 20 shows THP1-GFP PMA pre-treated cells exposed to TTFields for 24 hours;
Figure 21 shows THP1-GFP PMA pre-treated control cells not exposed to TTFields;
Figure 22 shows that TTFields induce pyroptosis-dependent Caspase-1 activation after exposure to TTFields for 1 day and 3 days;
Figure 23 shows that TTFields-induced caspase-1 activation and pyroptosis coincide with lower level of full-length IL-1 beta and higher LDH release after exposure to TTFields for 1 day and 3 days;
Figure 24 shows that TTFields-induced STING/AIM2 activation and inflammatory cytokine production persist for at least 3 days after TTFields treatment has ended;
Figure 25 shows that short pulsed TTF-induced STING/AIM2 activity is associated with reduced tumor growth and increased DC (dendritic cells) recruitment to deep cervical draining lymph nodes; and
Figure 26 shows that caspase 1 is detected in TTFields-treated cells without AIM2.

### DETAILED DESCRIPTION

Glioblastoma (GBM) is the most common and deadliest malignant brain cancer in adults despite aggressive chemoradiotherapy. Tumor Treating Fields (TTFields) was recently approved in combination with adjuvant temozolomide chemotherapy for newly diagnosed GBM patients. The addition of TTFields resulted in a significant improvement in overall survival. TTFields are low-intensity alternating electric fields that are thought to disturb mitotic macromolecules' assembly, leading to disrupted chromosomal segregation, integrity and stability. In many patients, a transient stage of increased peritumoral edema is often observed early in the course of TTFields treatment followed subsequently by objective radiographic responses, suggesting that a major component of therapeutic efficacy by TTFields may be an immune mediated process. However, the mechanism underlying these observations remains unclear.

As described herein, TTFields-activated micronuclei-dsDNA sensor complexes led to i) induction of pyroptotic cell death, as measured by a specific LDH release assay, and through AIM2-recruited caspase1 and cleavage of pyroptosis-specific Gasdermin D; and ii) activation of STING pathway components including Type I interferons (IFNs) and pro-inflammatory cytokines downstream of the NFκB pathway. *See, e.g.,* Figure 3. GBM cell-specific shRNA depletion of either AIM2 or STING or both in a co-culture experiment of bone marrow cells or splenocytes with supernatants obtained from knockdown GBM cells was able to reverse the inducement of immune cells.

GBM cell lines treated with TTFields at the clinically approved frequency of 200 kHz using an in vitro TTFields system. In one aspect, 24 hours TTFields-treated GBM cells had a significantly higher rate (19.9% vs. 4.3%, p=0.0032) of micronuclei structures released into the cytoplasm as a result of TTFields-induced chromosomal instability. Nearly 40% of these micronuclei were co-localized with two upstream dsDNA sensors (absent in melanoma 2 (AIM2) and Interferon (IFN)-inducible protein Cyclic GMP-AMP synthase (cGAS)) compared to absence of co-localization in untreated cells. These results demonstrate that TTFields activate the immune system in GBM cells.

Aspects described herein provide methods of reducing the viability of cancer cells by applying alternating electric fields to the cancer cells at a frequency between 100 and 500 kHz for t 3 to 10 days and administering a checkpoint inhibitor to the cancer cells. The alternating electric fields can be applied to the cancer cells continuously or discontinuously for 3 to 10 days. In another aspect, the alternating electric fields can be applied to the cancer cells for at least 4 hours per day on each of the 3 to 10 days, or at least 6 hours per day on each of the 3 to 10 days. Alternating electric fields can optionally be applied to the cancer cells for 3, 4, 5, 6, 7, 8, 9, 10, 11, 12, 13, 14, or 15 days. In another aspect, the alternating electric fields can be applied to the cancer cells for 3-5, 3-6, 3-7, 3-8, 3-9, or 3-15 days.

The term "reducing the viability of cancer cells" refers to shortening, limiting, or having a negative impact on the ability of cancer cell to remain alive. For example, reducing the rate of growth or reproduction of a cancer cell reduces its viability.

The term "administering a checkpoint inhibitor" refers to providing the checkpoint inhibitor to a patient by a healthcare professional or the patient through any suitable and accepted route of administration (e.g., oral, intravenous, parenteral, topical etc.) as approved on the product label by a regulatory authority, under the care of a healthcare professional, or as part of an approved clinical trial. Prescribing a checkpoint inhibitor can also be "administering" a checkpoint inhibitor.

The term "continuously" refers to applying alternating electric fields for a substantially constant period of time. Continuous application of alternating electric fields can occur even if the application is discontinued for a short period of time (e.g., seconds) in order to position equipment appropriately, or if there is a brief disruption of power.

The term "discontinuously" refers to applying alternating electric fields for a period of time with a periodic break or disruption for seconds, minutes, an hour or more. In this aspect, a patient could apply alternating electric fields for a period of time (e.g., 1, 2, 3, or 4 hours) with a 15 minute, 30 minute, 45 minute, 1 hour period without applying the alternating electric field. In another aspect, the patient could apply the alternating field continuously while sleeping and discontinuously while awake. In a further aspect, the patient can apply the alternating electric field continuously except during mealtime or during a social event.

In a further aspect, the alternating electric fields are applied to the cancer cells for at least 4 or 6 hours per day on each of the 3 to 10 days.

In yet another aspect, the alternating electric fields are applied to the cancer cells for 3 days, followed by a period of 3 days where the alternating electric fields are not applied to the cancer cells, followed by a period of 3 days where the alternating electric fields are applied to the cancer cells.

In another aspect, the alternating electric fields are applied to the cancer cells at least 3 days per week.

In a further aspect, the alternating electric fields are applied to the cancer cells for a first period of 3 to 10 days followed by a second period where the alternating electric fields are not applied. In another aspect, the second period is at least the same as the first period.

This aspect can significantly improve comfort and convenience for the patient because a device for applying TTFields can be worn by the patient during a period of time when the patient is at home or sleeping when it is more convenient to wear the device continuously. The patient does not have to continue to wear the device during a period of time when the patient would rather be unencumbered by a medical device (e.g., working, exercising, participating in social activities).

Thus, a patient will receive needed TTFields treatment followed by taking, for example, a pill for a checkpoint inhibitor without continuing to wear the device in public or social settings. Compliance with treatment will be improved along with comfort for the patient. Discontinuing use of TTFields during a treatment cycle as described herein has not been disclosed or suggested previously.

In yet another aspect, the alternating electric fields are applied to the cancer cells in short pulses. The term "short pulse" refers to a discontinuous alternating electric field applied to cancer cells where each pulse has a duration of, for example, less than 5 seconds.

The cancer cells can be selected from the group consisting of glioblastoma cells, pancreatic cancer cells, ovarian cancer cells, non-small cell lung cancer (NSCLC) cells, and mesothelioma. In a further aspect, the cancer cells are glioblastoma cells.

The checkpoint inhibitor can be selected, for example from the group consisting of ipilimumab, pembrolizumab, and nivolumab.

The alternating electric fields can have a frequency between 180 and 220 kHz.

In yet another aspect, at least a part of administering the checkpoint inhibitor to the cancer cells occurs after discontinuing applying the alternating electric fields to the cancer cells at a frequency between 100 and 500 kHz for the 3 to 10 days. In accordance with the invention, the administering the checkpoint inhibitor to the subject occurs after discontinuing applying alternating the electric fields.

Further aspects provide methods of treating glioblastoma by applying alternating electric fields to the head of a subject with glioblastoma at a frequency between 100 and 500 kHz for 3 days and administering a checkpoint inhibitor to the subject. The alternating electric fields are applied to the subject continuously or discontinuously for 3 days. In another aspect, the alternating electric fields are applied to the subject for at least 4 hours per day on each of the 3 days. The checkpoint inhibitor can be selected from the group consisting of ipilimumab, pembrolizumab, and nivolumab. The alternating electric fields can have a frequency between 180 and 220 kHz.

In a further aspect, at least a part of administering the checkpoint inhibitor to the subject occurs after discontinuing applying alternating the electric fields to the head of the subject with glioblastoma at a frequency between 100 and 500 kHz for the 3 to 10 days.

Further aspects provide methods of reducing viability of cancer cells comprising applying alternating electric fields to the cancer cells at a frequency between 100 and 500 kHz for a time sufficient to kill about 1-2% of the cancer cells; and administering a checkpoint inhibitor to the cancer cell. In one aspect, a time period sufficient to kill about 1-2% of the cancer cells is 3, 4, 5, 6, 7, 8, 9, or 10 days.

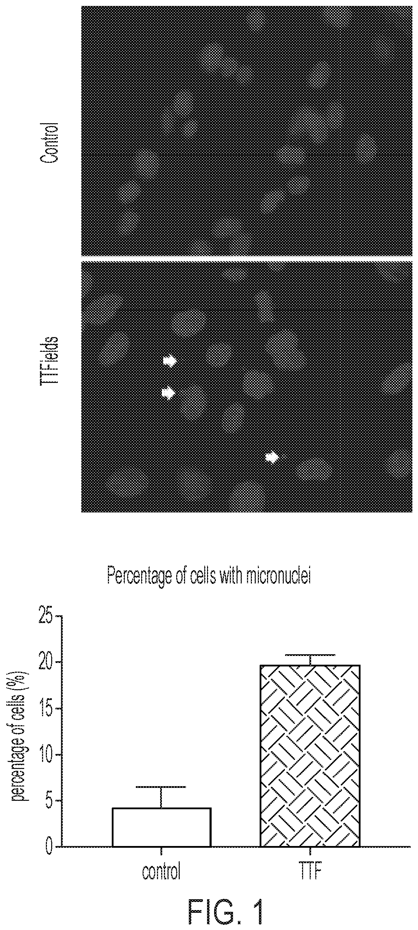

TTFields can induce the formation of cytoplasmic micronuclei GBM cells exposed to TTFields. Figure 1 shows the results of exemplary experiments where LN827 cells were treated by TTFields for 24 hours and then fixed by 4% PFA for 20 min. DAPI (4',6-diamidino-2-phenylindole) (1:5000) stain was incubated for 5 min at room temperature to stain for the nucleus and micronuclei. Figure 1 (right panel) shows the percentage of control cells with micronuclei (approximately 4%) vs. TTFields exposed cells (approximately 20%). Thus, TTFields induce the formation of cytoplasmic micronuclei (dsDNA) which can induce the STING pathway.

While small molecule STING activators (e.g., STING agonists) are known and are in clinical development (Ryan Cross, STING fever is sweeping through the cancer immunotherapy world, Volume 96 Issue 9 | pp. 24-26, Chemical & Engineering News (February 26, 2018)), these drugs may have significant side effects for patients. In contrast, TTFields have virtually no side effects and therefore present a safer and more comfortable alternative to small molecule STING activators.

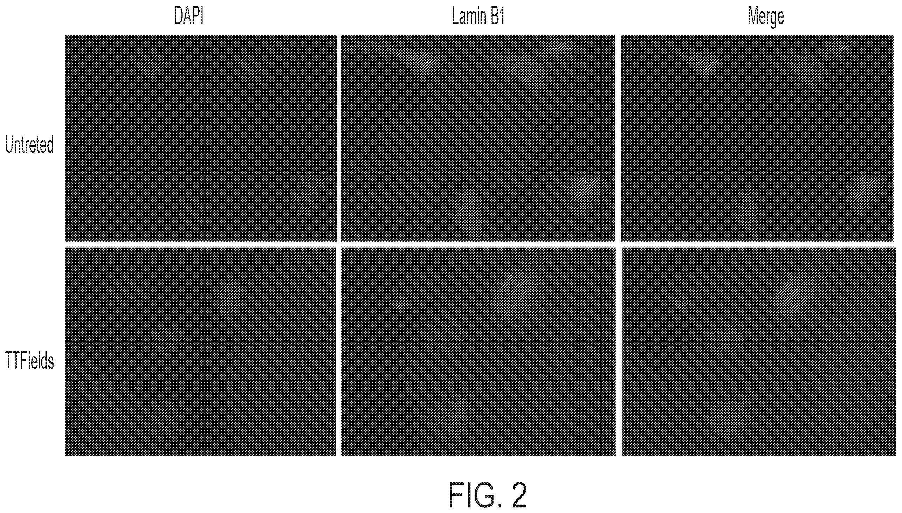

Lamin B1 structures are disrupted after exposure to TTFields, leading to release of dsDNA into the cytoplasm in LN827 cells. Figure 2 shows further nuclear disruption in LN827 cells treated by TTFields for 24 hours, fixed by 4% PFA for 20 min, and blocked by 0.2% Triton/0.04% BSA for 1 hour. DAPI stained cells are shown on the left side panels (TTFields untreated and treated as labelled). The middle panels show the results when cells were incubated with Lamin B1 antibody overnight at 4° C followed by incubation with a fluorescent secondary antibody for 1 hour (TTFields untreated and treated as labelled). The right side panels show the merged images (DAPI/Lamin B 1). These results indicate that dsDNA is released into the cytoplasm following TTFields application resulting in induction of the STING pathway.

Figure 3 depicts induction of the proinflammatory STING and pyroptosis pathways by dsDNA. dsDNA can be produced from micronuclei induced by abnormal mitosis. Abnormal mitosis can be induced, for example, by TTFields. TTFields can also reduce nuclear envelope integrity as shown by disruption of lamin B1 structures leading to dsDNA in the cytoplasm and induction of the STING pathway as shown.

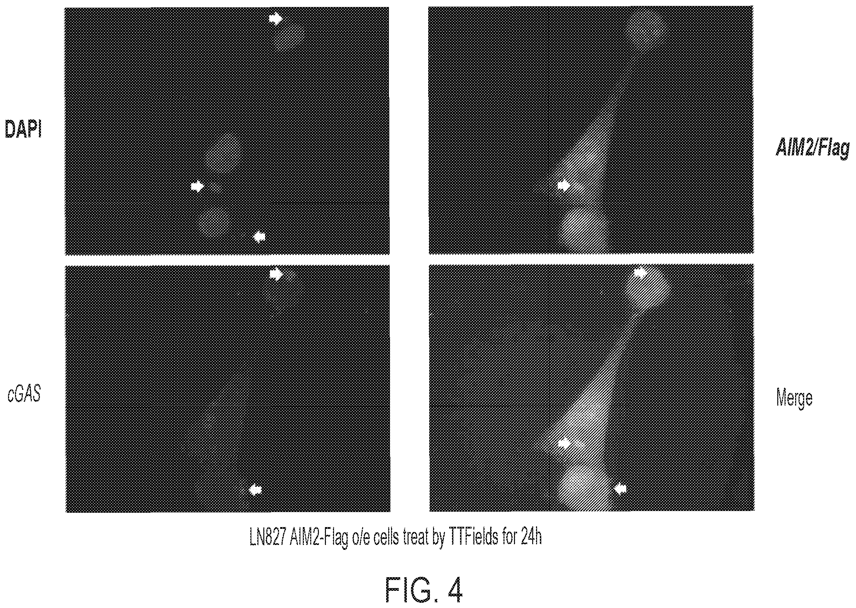

cGAS (Cyclic GMP-AMP synthase) and AIM2 independently co-localize with micronuclei in response to exposure to TTFields. cGAS and AIM2 are immune sensors that detect the presence of cytoplasmic dsDNA. In Figure 4, LN827 cells were treated by TTFields for 24 hours, fixed by 4% PFA for 20 min, and blocked by 0.2% Triton/0.04% BSA (bovine serum albumin) for 1 hour. Flag and cGAS antibodies were incubated over night at 4° C followed by incubation with secondary antibody for 1 hour, and DAPI staining for 5 minutes at room temperature.

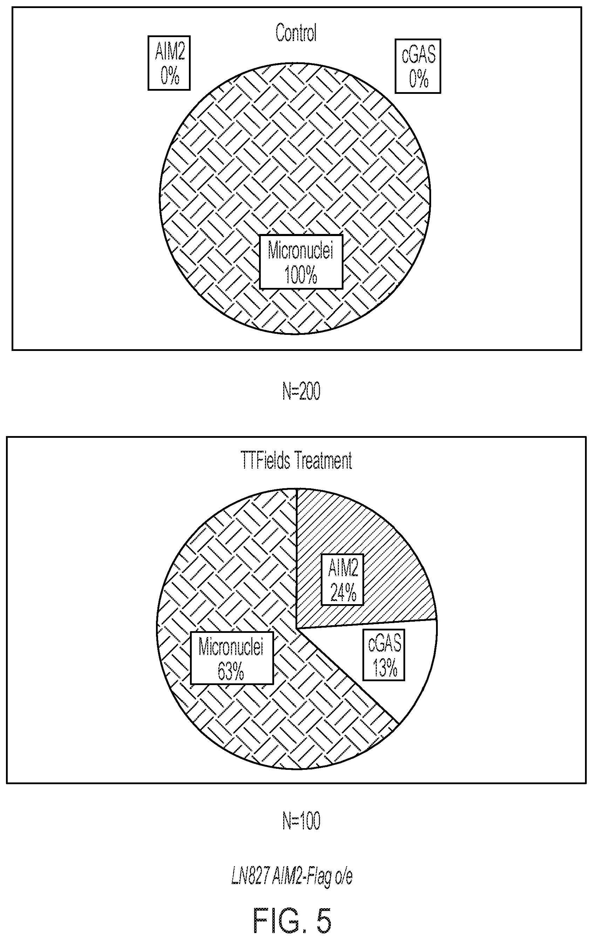

Thus, cGAS and AIM2 each independently co-localize with micronuclei in response to TTFields indicating that TTFields induces the presence of cytoplasmic dsDNA, activates the STING pathway. Figure 5 quantifies the percentages of cGAS, AIM2 and micronuclei from the results of Figure 4 with and without exposure to TTFields.

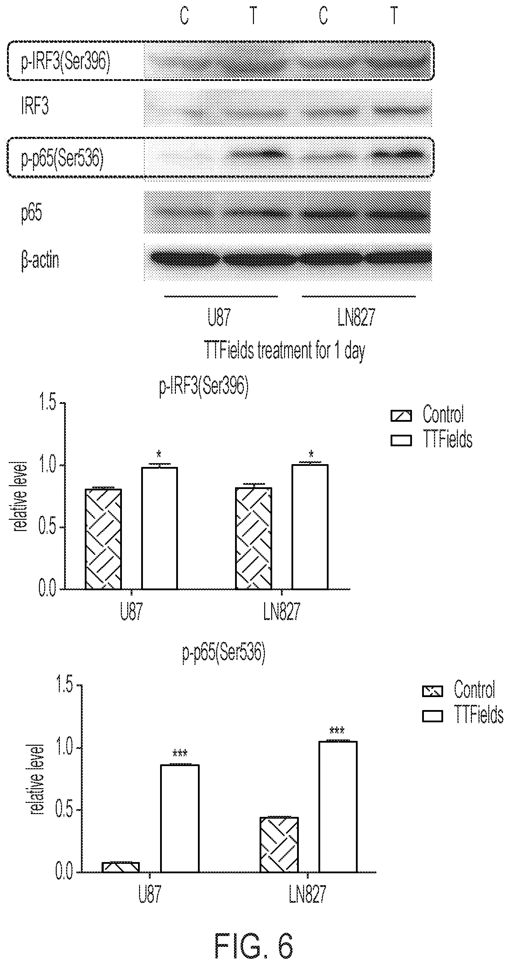

IRF3 and p65 are phosphorylated after exposure to TTFields for one day in U87 and LN827 cells. In Figure 6 , total protein was collected after U87 and LN827 cells were treated with TTFields for 24 hours. The presence of IRF3 and p65 downstream of STING pathway, as well as their activated phosphorylated forms, were measured by western blot. B-actin was used as a loading control. STING-induced Pathway (IRF3 and p65) is activated after TTFields as shown by the presence of the phosphorylated forms of IRF3 and p65. Thus, IRF3 or interferon regulatory factor 3 and p65 phosphorylation are triggered by STING activation.

TTFields induce Type I IFN response and pro-inflammatory cytokines downstream of STING. The term "downstream of STING" refers to cytokines that are induced following activation of the STING pathway. In this aspect, TTFields induce the STING response as described herein.

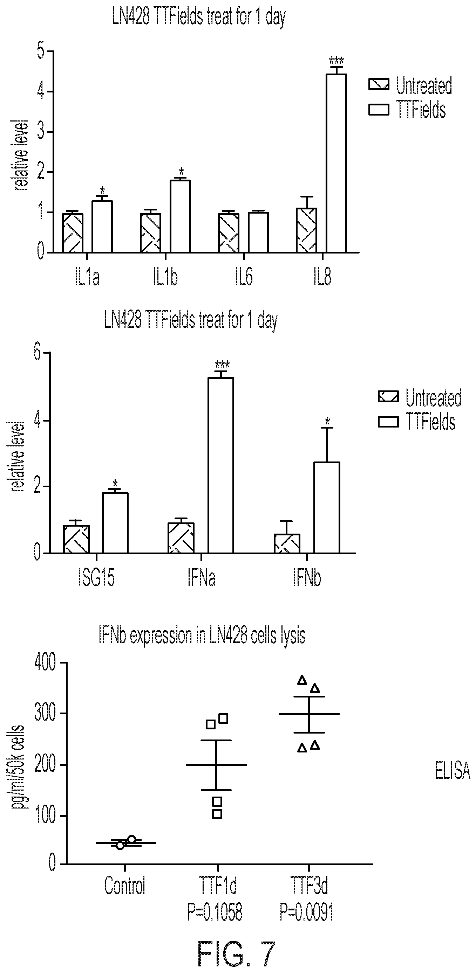

LN428 cells were treated for 24 hours with/without TTFields (Figure 7). Total RNA was extracted and converted into cDNA. Quantitative-PCR was utilized to detect the transcriptional levels of IL1α, IL1β, IL6, IL8 and ISG15, IFNα, IFNβ. Total LN428 protein was collected in protein lysis buffer and the cell number was determined. The endogenous protein level of IFNβ was determined by ELISA. The final protein level was normalized by cell number.

As shown in Figure 7, TTFields induces cytokines such as interferon b (IFNb) expression in LN428. In particular, exposure of LN428 cells to TTFields for 3 days increased IFNb levels 300 fold over the control and 100 fold over 1 day exposure to TTFields. In this aspect, applying TTFields for about 3 days significantly increased levels of pro-inflammatory cytokines.

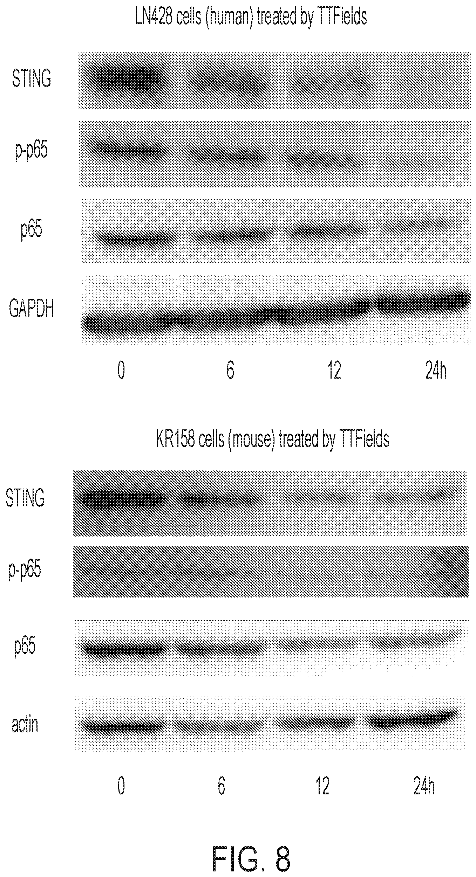

STING is degraded after becoming activated by TTFields in GBM cells. In the experiments summarized in Figure 8, LN428 (human) and KR158 (mouse) protein was collected at the indicated time points. STING, p65 and phosphorylated-p65 protein levels were determined by western blot. B-actin/GAPDH were used as loading controls. As shown in Figure 8, STING protein levels and phosphorylated-p65 levels are reduced over a 24 hour period of TTFields treatment.

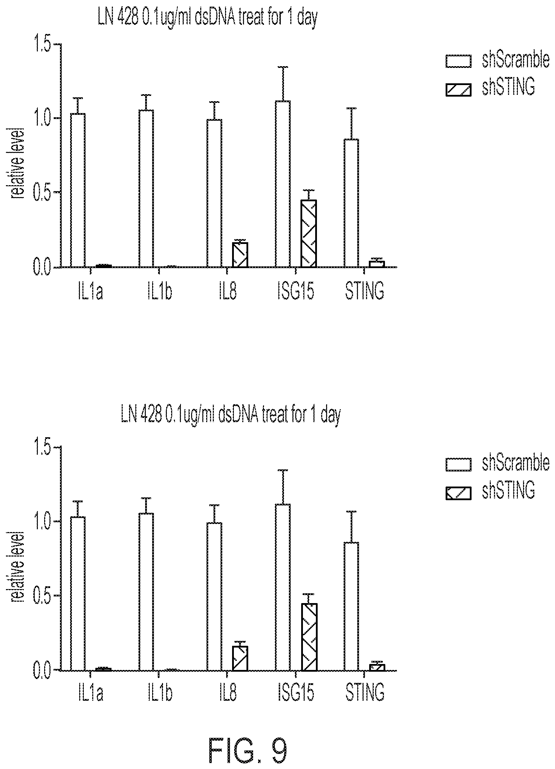

STING is required for inflammatory responses induced by dsDNA and TTFields treatment in human GBM cells (LN428 human cells). In the experiments summarized in Figure 9, human GBM cell line LN428 was stable infected by lentivirus-shScramble or shSTING. Cells were separately treated for 24 hours with dsDNA or TTFields. Polyethylenimine (PEI) was utilized as the transfection buffer to induce dsDNA migration into the cytoplasm. Total RNA was extracted and converted into cDNA. Quantitative-PCR was utilized to detect the transcriptional levels of IL1α, IL1β, IL8, ISG15 and STING.

As shown in Figure 9, levels of various cytokine RNA transcripts are reduced in the absence of STING (shSTING) when the STING pathway is induced by both dsDNA and TTFields in LN428 cells.

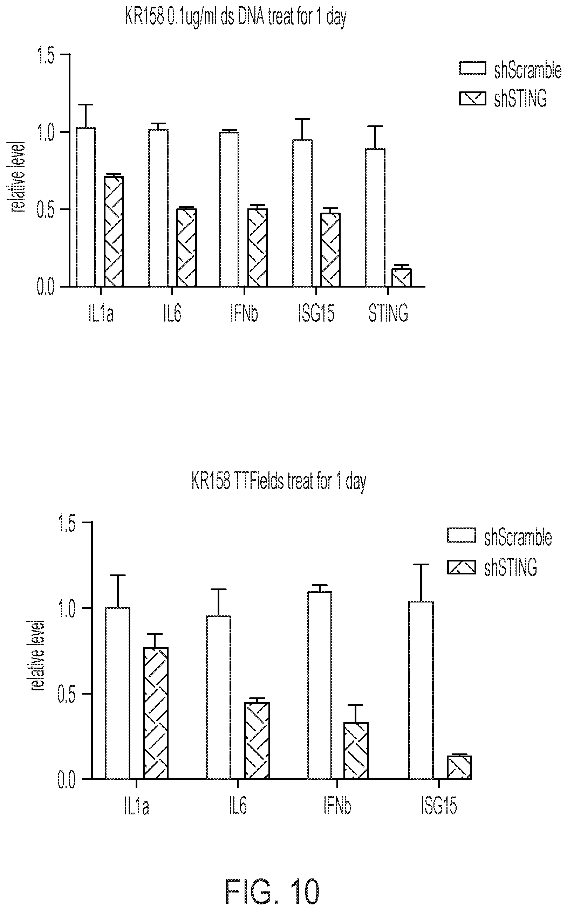

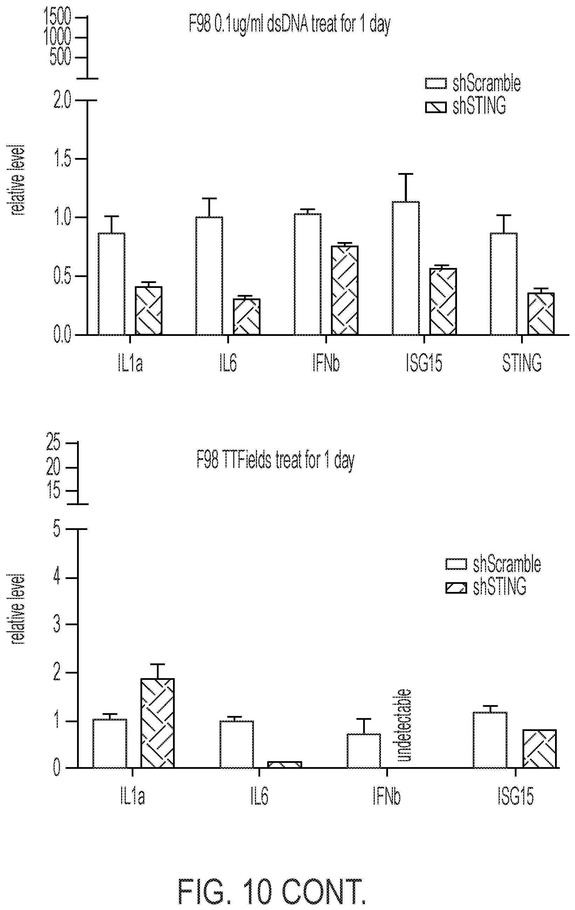

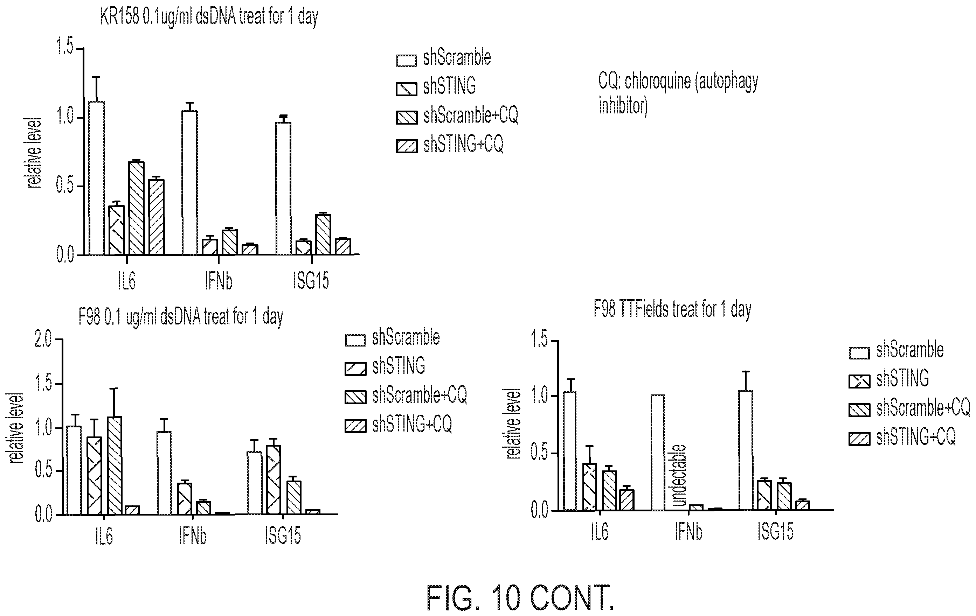

Autophagy and dsDNA or TTFields synergistically induce STING-dependent proinflammatory responses in KR158 and F98 GBM cells. In the experiments summarized in Figure 10, mouse GBM cell line KR158 and rat GBM cell line F98 were stably infected by lentivirus-shScramble or shSTING. Cells were separated and treated for 24 hours with dsDNA or TTFields. PEI was utilized as the transfection buffer to induce dsDNA into cytoplasm. Total RNA was extracted and converted into cDNA. Quantitative-PCR was utilized to detect the transcriptional levels of IL1α, IL6, ISG15, IFNβ and STING.

In a related experiment, cells as described above were separated and treated for 24 hours with/without the present of chloroquine (an autophagy inhibitor) and with dsDNA or TTFields. PEI was utilized as the transfection buffer to induce dsDNA into cytoplasm. Total RNA was extracted and converted into cDNA. Quantitative-PCR was utilized to detect the transcriptional levels of IL6, ISG15, IFNβ.

As shown in Figure 10, levels of various cytokine RNA transcripts are reduced in the absence of STING (shSTING) when the STING pathway is induced by both dsDNA and TTFields in KR158 and F98 cells. Levels of cytokine transcripts were further reduced by autophagy inducer coenzyme Q (CQ).

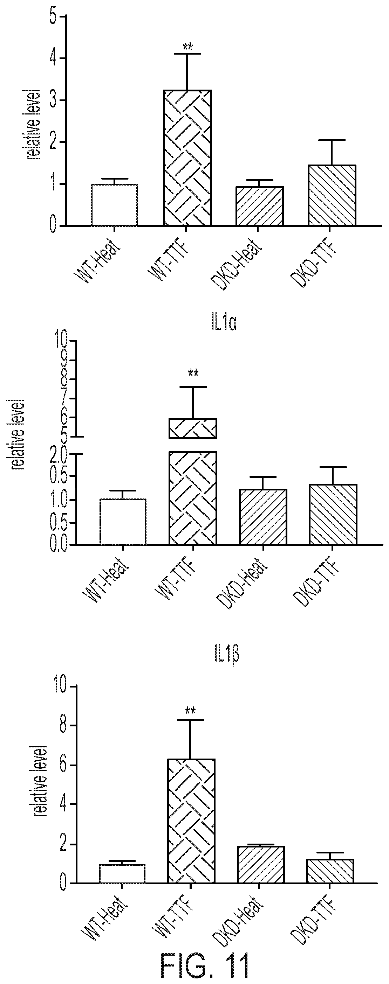

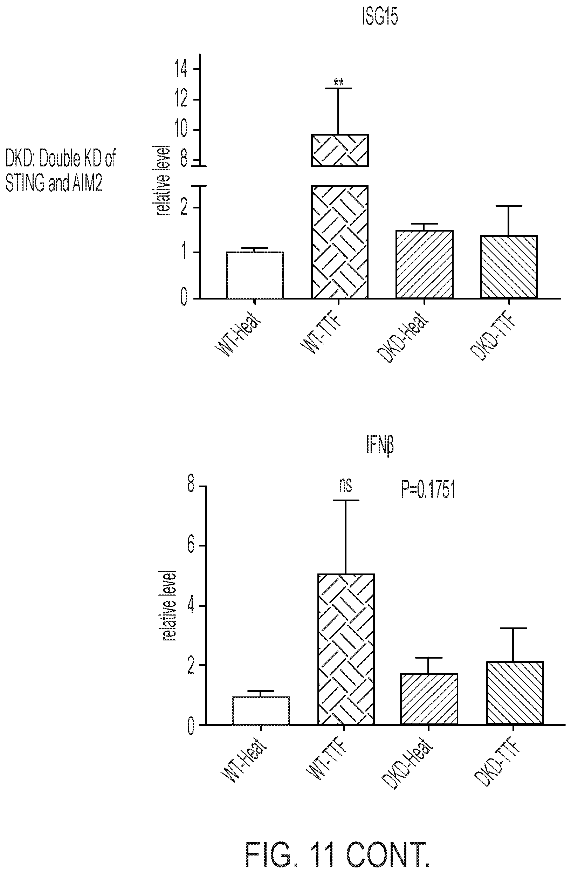

TTFields-induced inflammatory cytokine production is dependent on STING and AIM2 in the F98 Rat Glioma Model. In the experiments summarized in Figure 11, rat GBM cell line F98 was stable infected by lentivirus- scramble control (WT) or double knock down of STING and AIM2 (DKD). Cells were injected into the brains of male Fischer rats using a stereotaxis system. Seven days after the cells were injected, heat or TTFields was applied to the rats for additional 7 days. By end of the treatment, the rats were sacrificed, the tissues were collected and further analyzed. Quantitative-PCR was utilized to detect the transcriptional levels of IL1α, IL1β, IL6, ISG15 and IFNβ.

As shown in Figure 11, the double knock down of STING and AIM2 (DKD) significantly reduced the levels of the indicated cytokines.

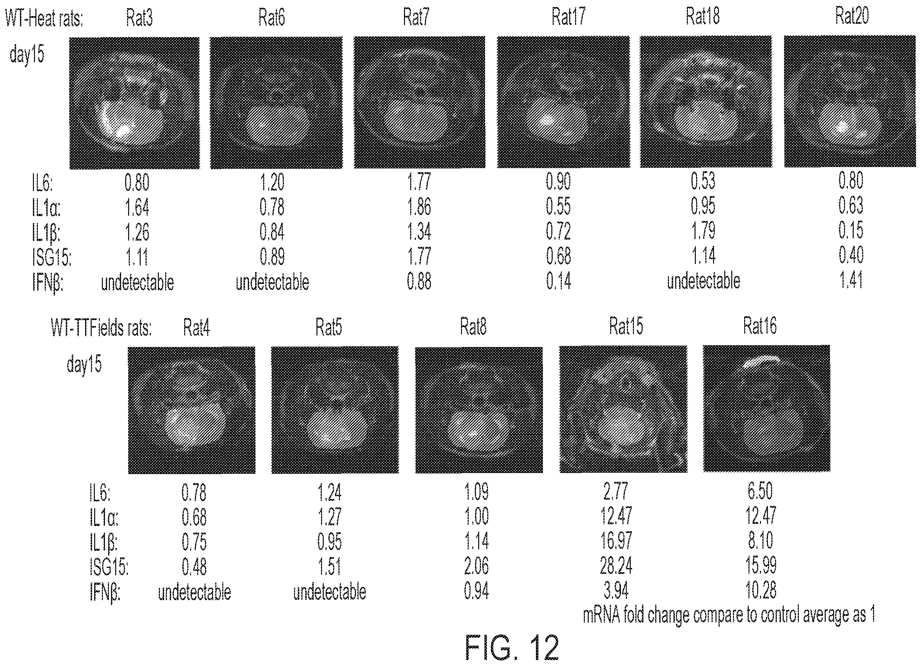

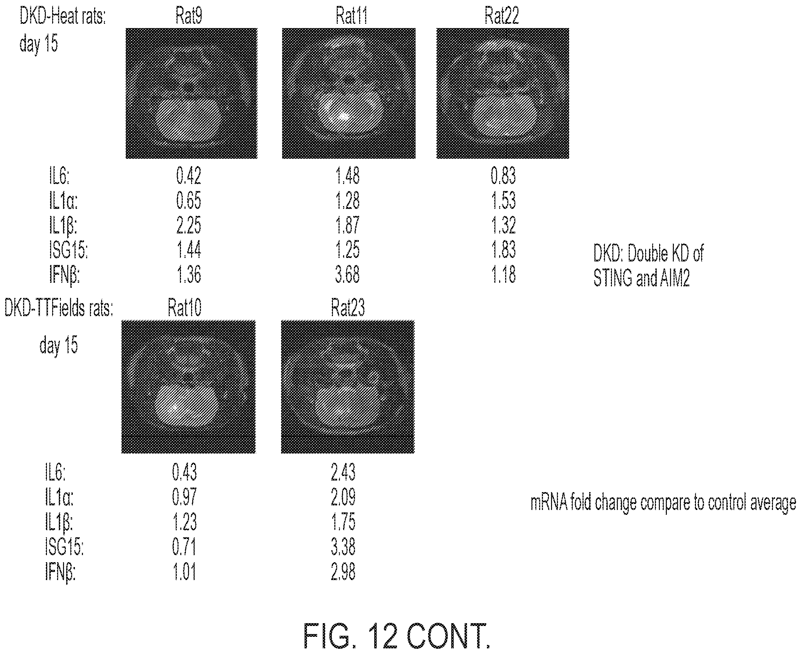

Tumor size is corelated with fold changes in inflammatory cytokine expression in response to TTFields. Figure 12 shows images of the rat brains utilized in the experiments summarized in Figure 11 The Quantitative-PCR results from Figure 11 (i.e., the relative mRNA levels of each individual rat's MRI picture) is shown below each picture on day 15 post-injection.

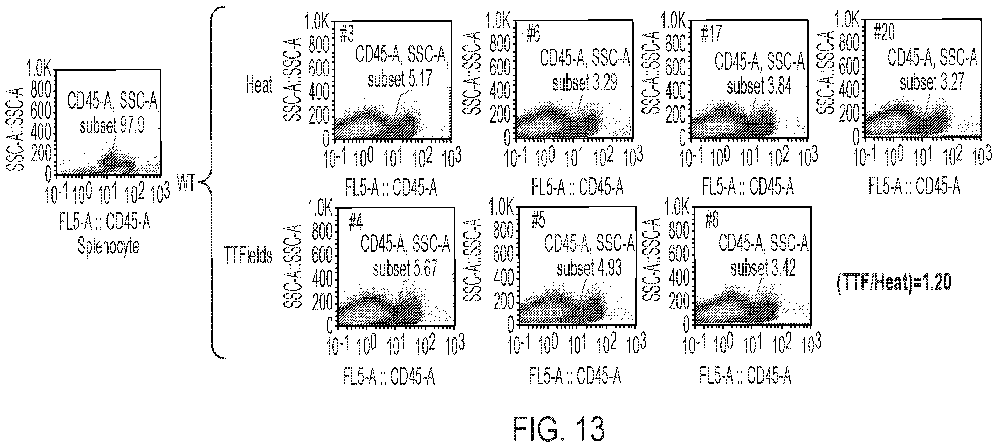

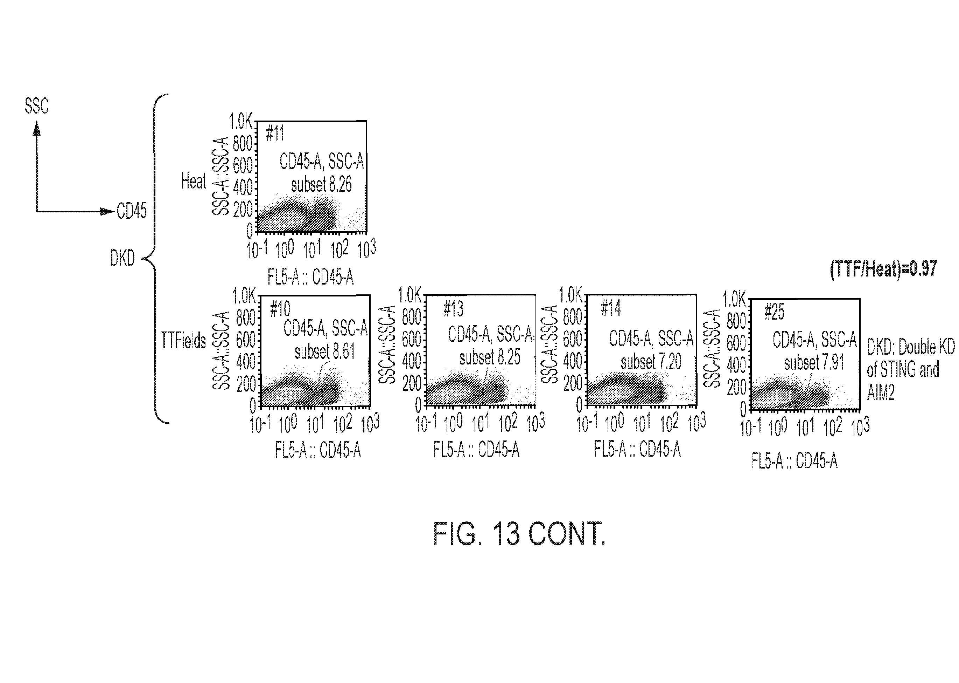

Figure 13 provides exemplary heat maps showing that recruitment of CD45 cells into GBM is lower in GBM lacking STING and AIM2 in the F98 Rat glioma model. In the experiment summarized in Figure 13, the rat GBM cell line F98 was stable edited by lentivirus- scramble control (WT) or double knock down of STING and AIM2 (DKD). Cells were injected into the brains of male Fischer rats using a stereotaxis system. 7 days after cell injection, heat or TTFields were applied to the rats for additional 7 days.

By end of the treatment, the rats were sacrificed, the tissues were collected, and split for further analysis. Here, the bulk tumors were dissociated into single cell suspensions. Multiple flow antibodies were used to stain for CD45. Then, the single cell suspension was fixed and analyzed on a flow cytometry machine on the following day.

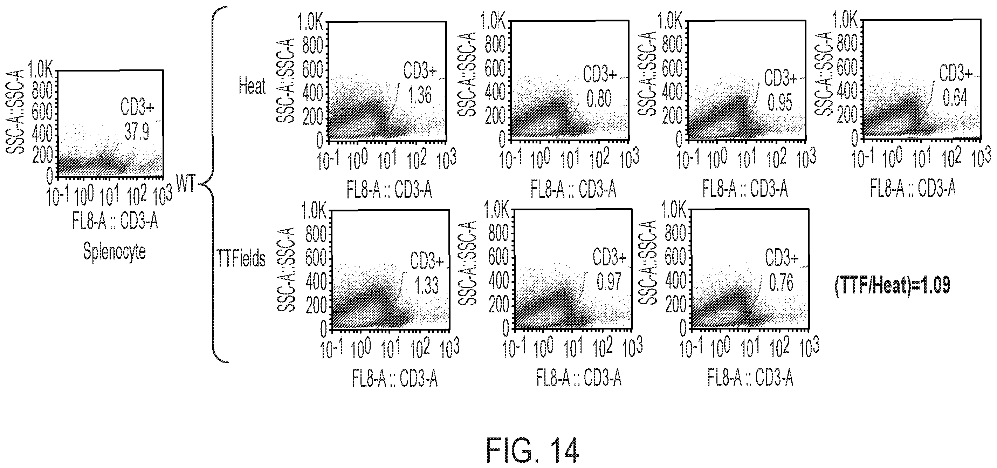

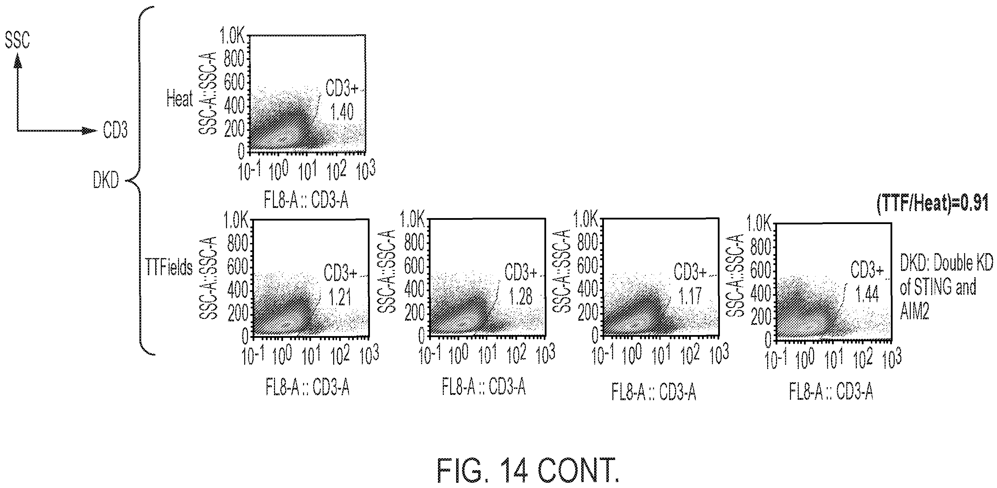

Figure 14 provides exemplary heat maps showing that CD3 (T cells) recruitment is lower in GBM lacking STING and AIM2. Figure 14 summarizes the same experiment as Figure 13 but using antibodies for CD3.

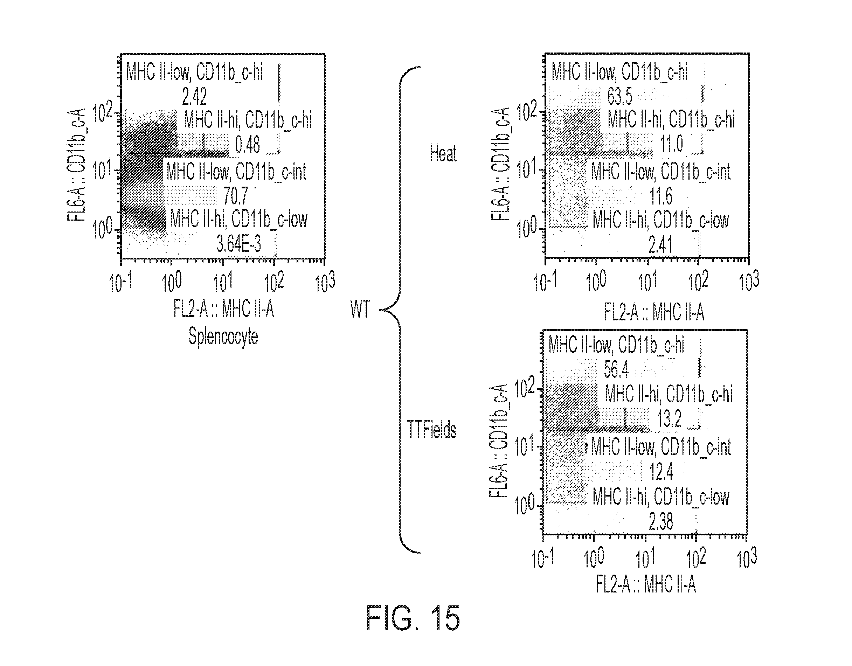

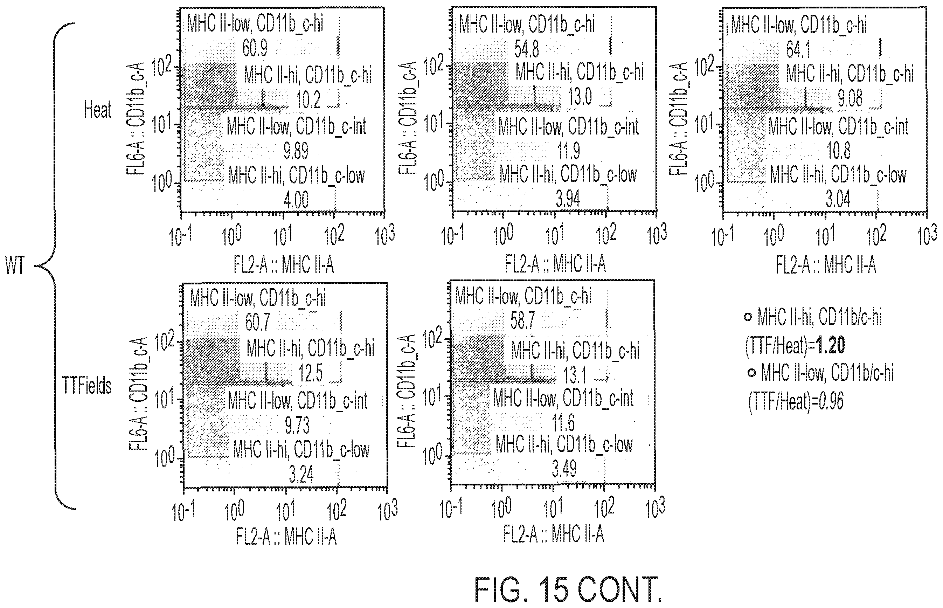

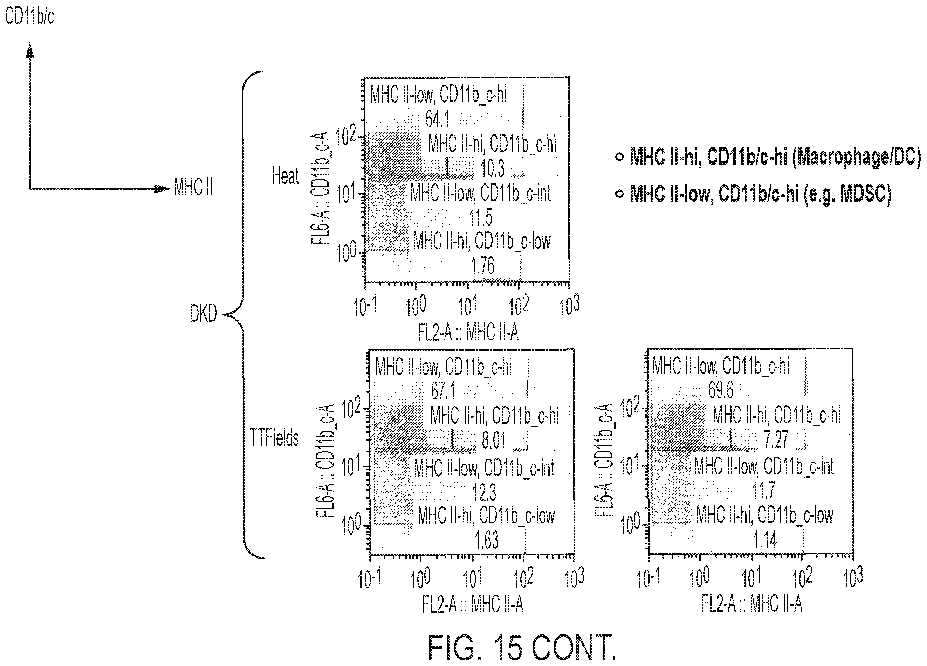

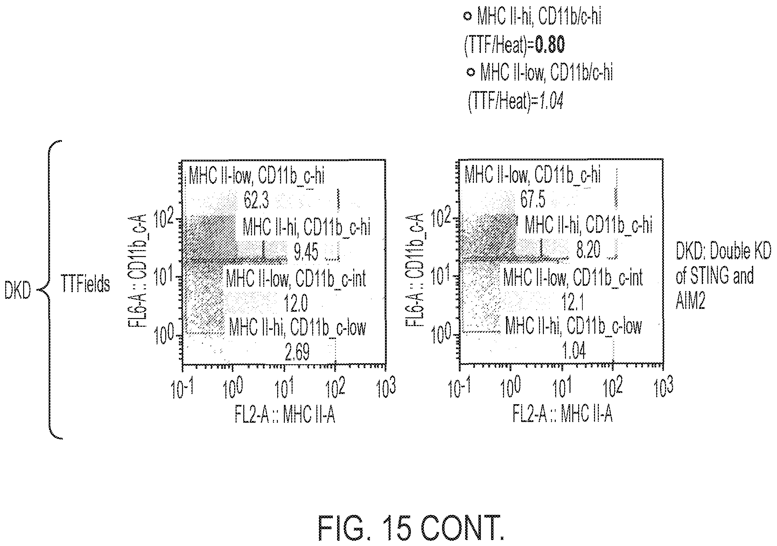

Figure 15 provides exemplary heat maps showing that in GBM lacking STING and AIM2, DC/Macrophage recruitment is lower and MDSC recruitment is higher. Figure 15 summarizes the same experiment as Figure 13 with antibodies directed to detecting CD11b/c and MHC II (macrophages).

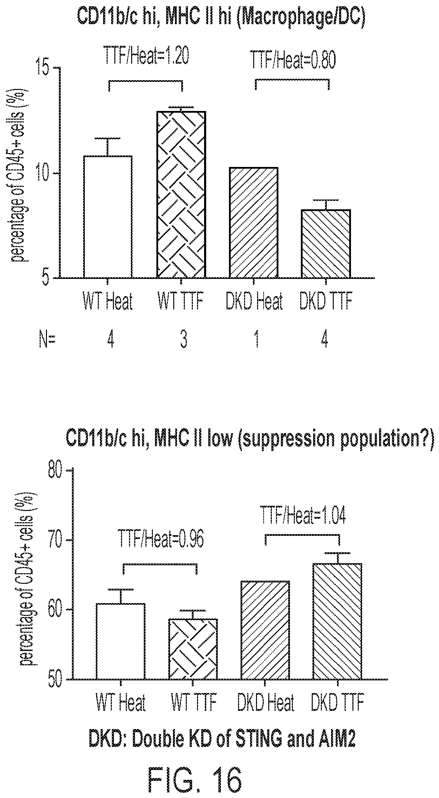

Figure 16 provides quantitative data from the flow cytometry results of Figure 15.

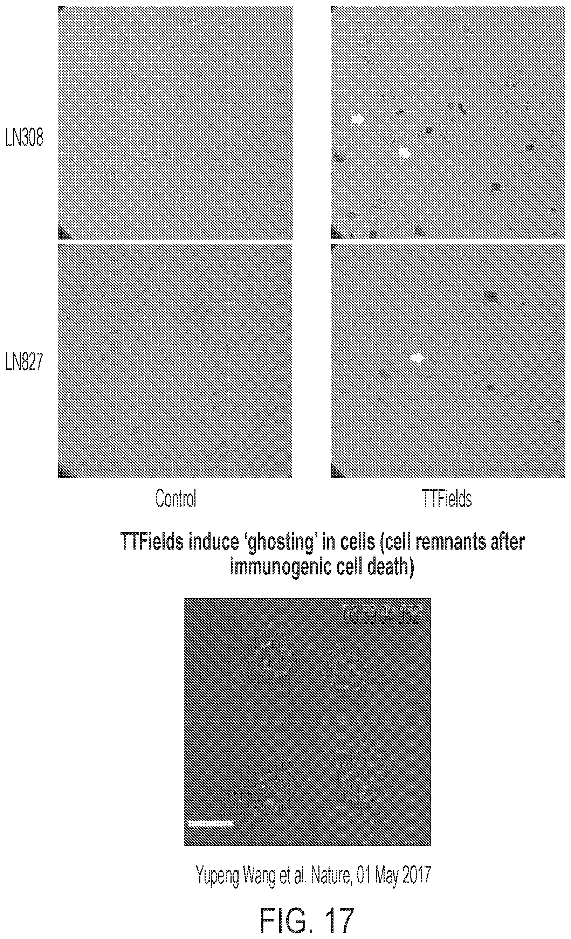

Figure 17 shows 'ghosting' induced by three days of exposure to TTFields in human GBM cell lines LN308 and LN827 Human GBM cell lines. The term "ghosting" refers to the presence of cell remnants that remain after immunogenic cell death. In these experiments, LN308 and LN827 cells were treated for 3 days with/without TTFields. Images were taken under bright field microscope. The images show increased immunogenic cell death following 3 days of TTFields exposure.

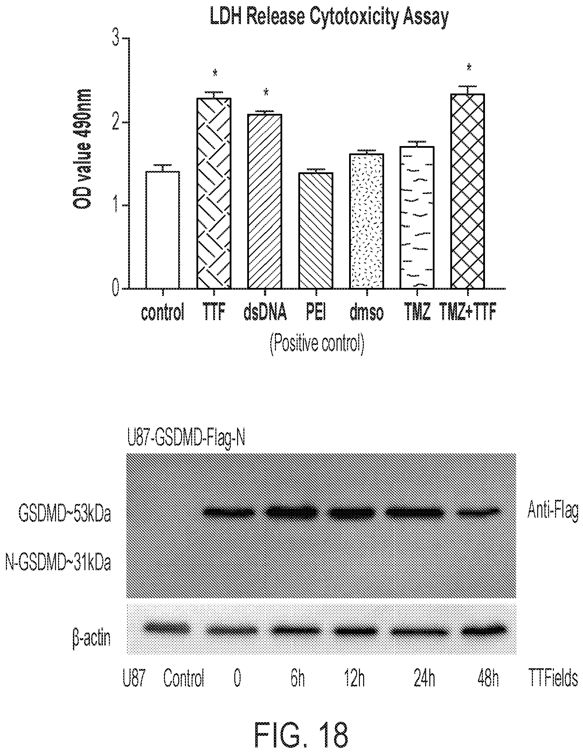

Figure 18 shows that TTFields induce membrane damage and decrease GSDMD in U87 GBM cells exposed to TTFields. In the experiment summarized in Figure 18, human GBM cell line U87 was treated under the indicated conditions for 24 hours. Lactic Acid Dehydrogenase (LDH) released into cell culture medium was detected by a cytotoxicity assay. U87 cells were forced to express Gasdermin D (GSDMD) by lentivirus-GSDMD-Flag-N and treated with/without TTFields. Total protein was collected as indicated time points. Overexpressed protein GSDMD levels were determined by western blot using flag antibody. B-actin was used as a loading control. As shown in Figure 18, exposure to TTFields killed 1-2% of the cells.

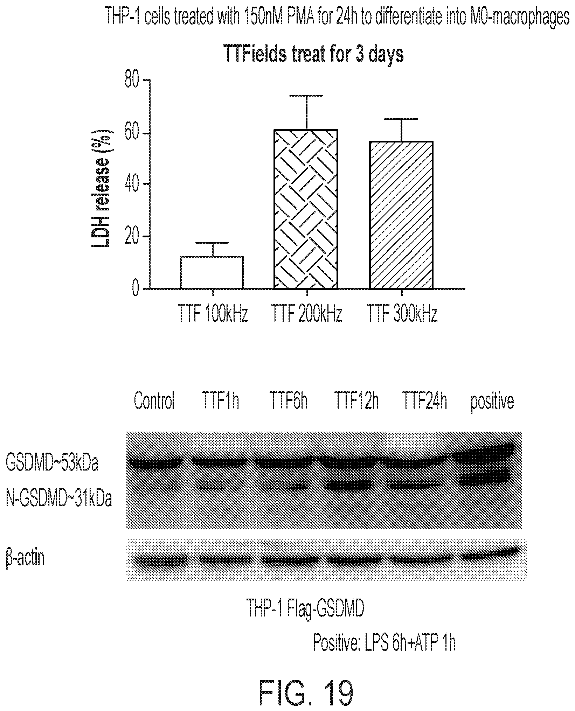

Figure 19 shows that TTFields induce membrane damage and cleaves GSDMD in human leukemia monocyte cell line THP-1 macrophages. In the experiment summarized in Figure 19, human leukemia monocyte cell line THP-1 was treated with 150nM PMA treatment for 24 hours to stimulate differentiation into macrophages. LDH release was tested on day 3 of TTFields treatment to examine electric field frequency ranges. THP-1 cells were forced to express GSDMD by lentivirus-GSDMD-Flag-N, and treated with/without TTFields. Total protein was collected as indicated time points. Overexpressed protein GSDMD levels and its cleaved N-fragments were determined by western blot using flag antibody. B-actin was used as a loading control. The positive control is shown as LPS treatment for 6 hours followed by 1 hour ATP.

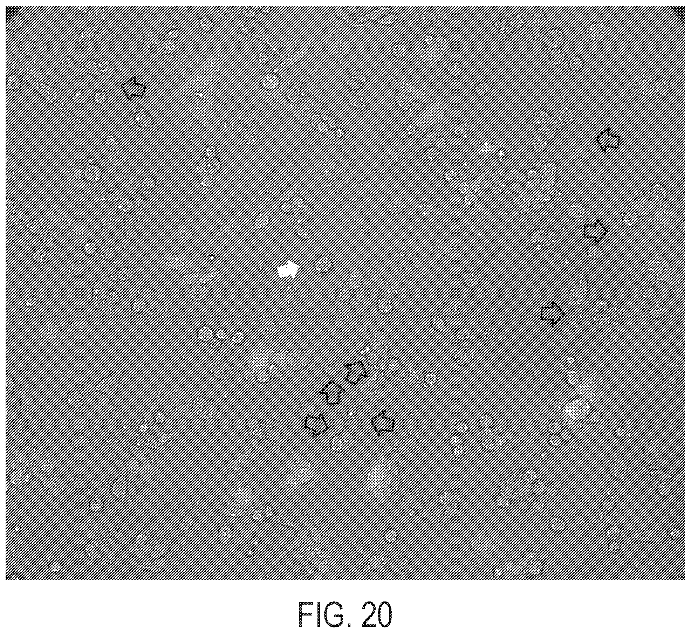

Figure 20 shows THP-1 cells labeled by GFP lentivirus and pre-treated by 150nM PMA for 24 hours. After a 24 hour period, cells were exposed to TTFields for 24 hours, and a time course image was captured every 20 minutes.

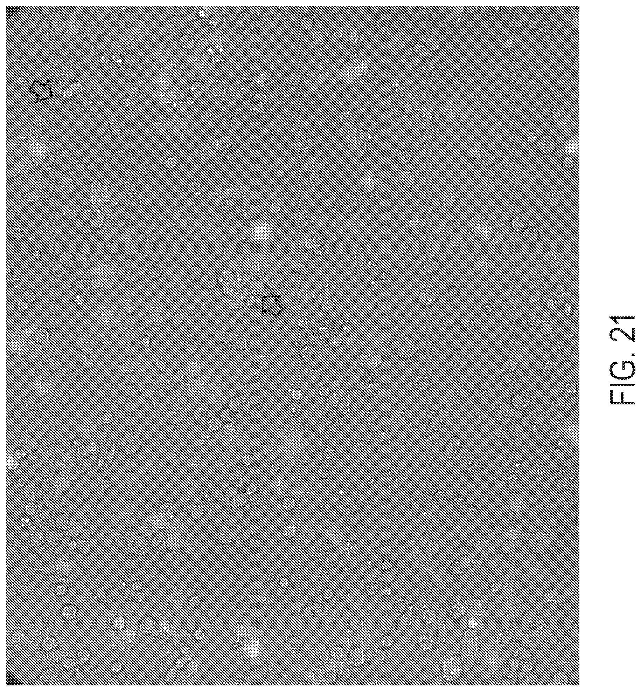

Figure 21 shows THP-1 cells labeled by GFP lentivirus and pre-treated by 150nM PMA for 24 hours. After a 24 hour period, cells were grown in normal culture conditions for 24 hours, and a time course image was captured every 20 minutes.

As shown in Figures 20 and 21, treatment with TTFields results in greater immunogenic cell death (Figure 20) compared to the control cells (Figure 21).

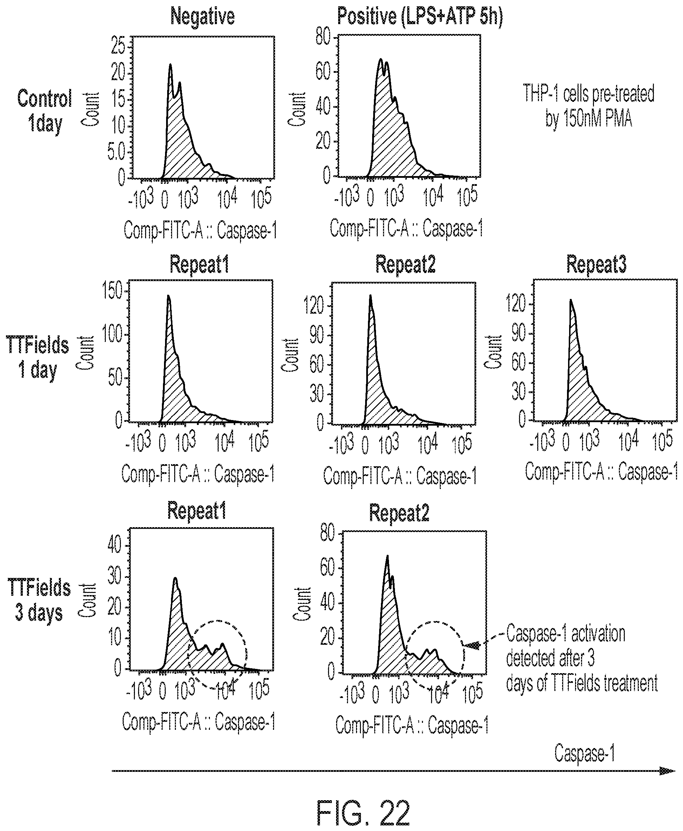

Figure 22 shows that TTFields induce pyroptosis-dependent Caspase-1 activation after exposure to TTFields for 1 day and 3 days. In the experiment summarized in Figure 22, THP-1 cells were pre-treated with 150nM PMA for 24 hours. After a 24 hour period, cells were treated with and without TTFields for the indicated time points. A caspase-1 activation detection kit was used to label the cells having cleaved caspase-1 form. The samples were analyzed on a flow cytometry machine.

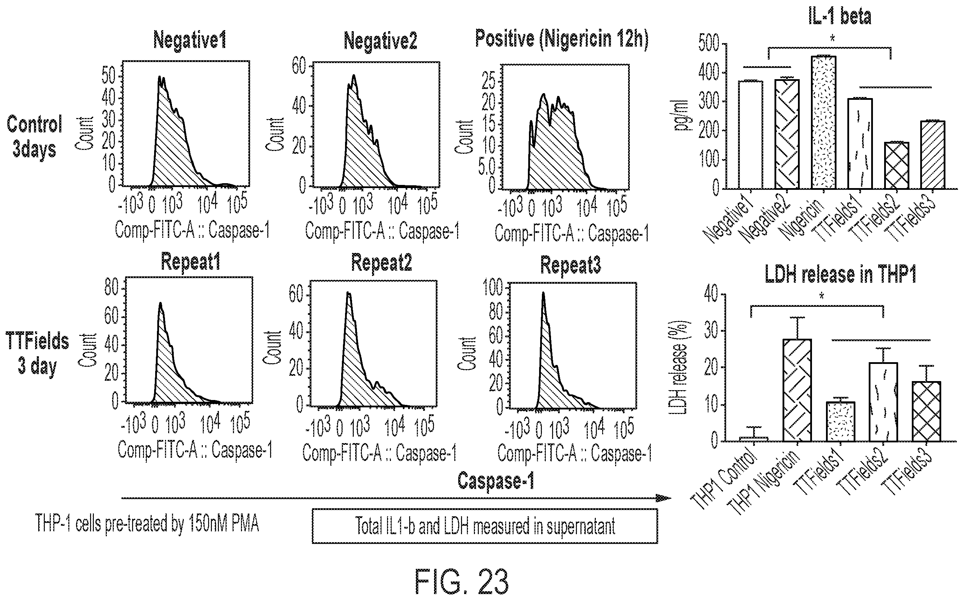

Figure 23 shows that TTFields-induced caspase-1 activation and pyroptosis coincide with lower levels of full-length IL-1 beta, and higher LDH release levels after exposure to TTFields for 1 day and 3 days. In the experiment summarized in Figure 23, THP-1 cells were pre-treated with 150nM PMA for 24 hours. After a 24 hour period, cells were treated with and without TTFields for 3 days. Nigericin was utilized for 12 hours as a positive control. A caspase-1 activation detection kit was used to label the cells having cleaved caspase-1 form. The samples were analyzed on a flow cytometry machine. The cell culture medium was collected at the same time point on day 3. IL1β and LDH levels in culture medium were determined by ELISA and a cytotoxicity assay.

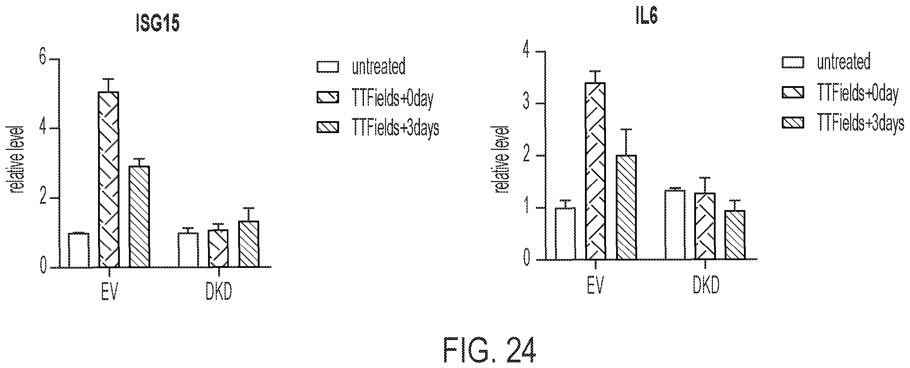

Figure 24 shows that TTFields-induced STING/AIM2 activation and inflammatory cytokine production persist for at least 3 days after TTFields treatment has ended. As shown in Figure 24, inflammatory cytokine production induced by TTF is dependent on STING and AIM2 and remains elevated above baseline several days after a brief pulse of TTF treatment. In the experiment summarized in Figure 24, K-LUC cells were transduced with an empty virus or virus carrying double-stranded shRNA targeting and inhibiting STING and AIM2. 30k of these cells per dish were then treated with TTFields for 3 days, then cultured for additional 3 days after TTF withdrawal and collected for inflammatory cytokine determination (IL-6 and ISG15). As shown in Figure 24, elevated IL6 and ISG15 production continued for at least 3 days after TTFields were discontinued (blue bar, EV).

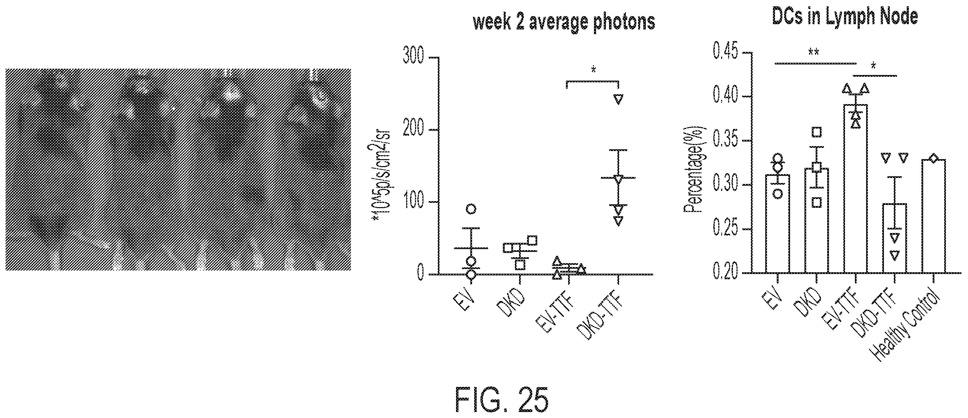

Figure 25 shows that short pulsed TTF-induced STING/AIM2 activity is associated with reduced tumor growth and increased DC (dendritic cells) recruitment to deep cervical draining lymph nodes. This persistent inflammatory activation by TTFields through STING/AIM2 activated the immune system after TTFields treatment was withdrawn.

In the experiment summarized in Figure 25, K-LUC cells were transduced with empty virus or virus expressing double shRNA targeting STING and AIM2, then untreated or treated with TTF for 3 days. 3x10⁵ of these K-LUC cells were implanted orthotopically into B6 mice. Tumor growth was measured by luc BLI. As shown in the middle panel, tumor size after two weeks following implantation was greatly reduced in the empty virus (EV) TTFields mice compared to the double knock down (DKD) mice. These mice also exhibited the highest level of DC cells (e.g., T cells)(right panel). Deep cervical lymph nodes are thought to be where DC recruitment and priming of naive T cells occurs for antigens coming from the brain.

Thus, TTFields stimulate the immune system to produce an anti-tumor immune reaction, analogous to an in situ "vaccination" where cells are primed for further cancer therapy (e.g., TTFields treatment for at least three days followed by treatment with a checkpoint inhibitor, as defined by the present invention).

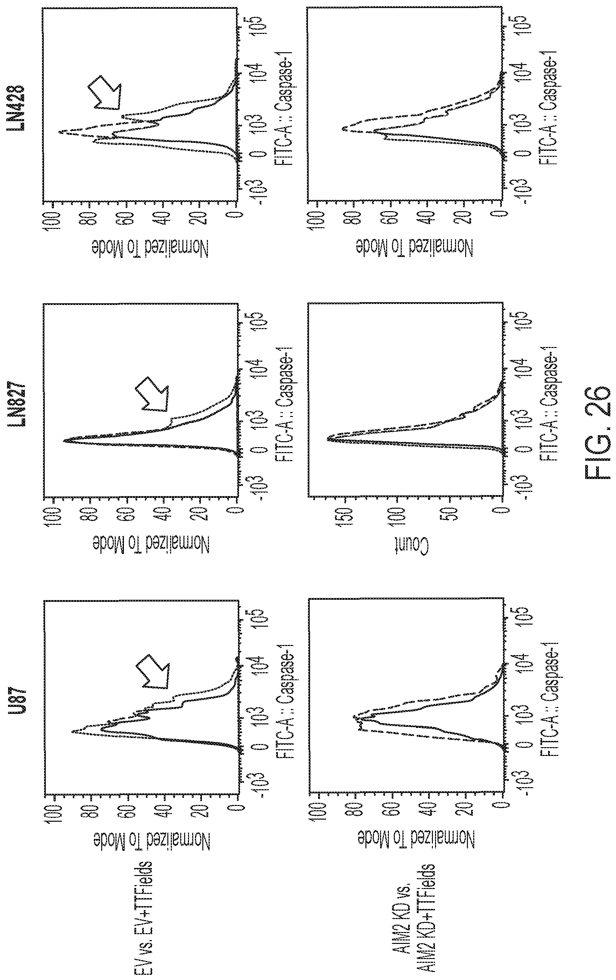

Figure 26 shows that caspase 1 is not detected in TTFields-treated cells without AIM2. Caspase 1 is immediately downstream of the AIM2-double stranded DNA complex and a molecular hallmark of pyroptosis. Caspase 1 activation is detected using a commercially available kit that detects the cleaved product (activated) of caspase 1. Activated caspase 1 is detected as a second FITC peak of the blue curve shifting to the right that was present only in TTFields-treated cells with normal AIM2 level (EV (empty virus) vs EV +TTFields). However, no such peak was observed in TTFields-treated cells without AIM2 (AIM2 KD vs. AIM2 KD+TTFields). Thus, the effects of TTFields on pyroptosis are mediated, at least in part, by AIM2.

The invention is defined by the appended claims. While the present invention has been disclosed with reference to certain embodiments, numerous modifications, alterations, and changes to the described embodiments are possible without departing from the scope of the present invention, as defined in the appended claims. Accordingly, it is intended that the present invention be defined in accordance with the appended claims.

## Claims

1. A checkpoint inhibitor for use in a method of treating cancer in a subject by reducing viability of cancer cells, the method comprising:
applying alternating electric fields to the cancer cells at a frequency between 100 and 500 kHz for at least 3 days; and administering the checkpoint inhibitor to the subject,
wherein the administering the checkpoint inhibitor to the subject occurs after discontinuing applying the alternating electric fields.

2. The checkpoint inhibitor for use according to claim 1, wherein the alternating electric fields are applied for 3 to 15 days, optionally wherein the alternating electric fields are applied for 3 to 10 days

3. The checkpoint inhibitor for use according to claim 1 or claim 2, wherein the alternating electric fields are applied to the cancer cells for at least 6 hours per day on each of the days.

4. The checkpoint inhibitor for use according to claim 1, wherein the alternating electric fields are applied to the cancer cells for 3 days, followed by a period of 3 days where the alternating electric fields are not applied to the cancer cells, followed by a period of 3 days where the alternating electric fields are applied to the cancer cells.

5. The checkpoint inhibitor for use according to claim 1, wherein the alternating electric fields are applied to the cancer cells at least 3 days per week.

6. The checkpoint inhibitor for use according to claim 1, wherein the alternating electric fields are applied to the cancer cells for a first period of at least 3 days followed by a second period where the alternating electric fields are not applied.

7. The checkpoint inhibitor for use according to claim 6, wherein the second period is at least the same as the first period.

8. The checkpoint inhibitor for use according to any preceding claim, wherein the alternating electric fields are applied to the cancer cells in short pulses.

9. The checkpoint inhibitor for use according to claim 1, wherein the cancer cells are selected from the group consisting of glioblastoma cells, pancreatic cancer cells, ovarian cancer cells, non-small cell lung cancer (NSCLC) cells, and mesothelioma.

10. The checkpoint inhibitor for use according to claim 1, wherein the cancer cells are glioblastoma cells.

11. A checkpoint inhibitor for use in a method of treating glioblastoma, the method comprising:
applying alternating electric fields to a head of a subject with glioblastoma at a frequency between 100 and 500 kHz for at least 3 days; and
administering the checkpoint inhibitor to the subject, wherein the administering the checkpoint inhibitor to the subject occurs after discontinuing applying the alternating electric fields.

12. The checkpoint inhibitor for use according to claim 11, wherein the alternating electric fields are applied for 3 to 15 days.

13. The checkpoint inhibitor for use according to claim 11, wherein the alternating electric fields are applied for 3 to 10 days.

14. The checkpoint inhibitor for use according to any of claims 11 to 13, wherein the alternating electric fields are applied to the subject continuously.

15. The checkpoint inhibitor for use according to any of claims 11 to 13, wherein the alternating electric fields are applied to subject discontinuously.

16. The checkpoint inhibitor for use according to claim 15, wherein the alternating electric fields are applied to the subject for at least 4 hours per day on each of the days.

17. The checkpoint inhibitor for use according to any of claims 11 to 16, wherein the checkpoint inhibitor is selected from the group consisting of ipilimumab, pembrolizumab, and nivolumab.

18. The checkpoint inhibitor for use according to any of claims 11 to 17, wherein the alternating electric fields have a frequency between 180 and 220 kHz.

19. The checkpoint inhibitor for use according to claim 11, wherein the alternating electric fields are applied to the head of the subject with glioblastoma for 3 days, followed by a period of 3 days where the alternating electric fields are not applied to the head of the subject with glioblastoma, followed by a period of 3 days where the alternating electric fields are applied to the head of the subject with glioblastoma.

20. The checkpoint inhibitor for use according to claim 11, wherein the alternating electric fields are applied to the head of the subject with glioblastoma in short pulses.

21. The checkpoint inhibitor for use according to claim 11, wherein the alternating electric fields are applied to the head of the subject with glioblastoma at least 3 days per week.

## Patentansprüche

1. Checkpoint-Inhibitor zur Verwendung in einem Verfahren zum Behandeln von Krebs bei einer Zielperson durch Verringern der Lebensfähigkeit von Krebszellen, wobei das Verfahren Folgendes umfasst:
Anlegen von elektrischen Wechselfeldern mit einer Frequenz zwischen 100 und 500 kHz an die Krebszellen für mindestens 3 Tage; und Verabreichen des Checkpoint-Inhibitors an die Zielperson,
wobei das Verabreichen des Checkpoint-Inhibitors an die Zielperson nach Beendigung des Anlegens der elektrischen Wechselfelder erfolgt.

2. Checkpoint-Inhibitor zur Verwendung nach Anspruch 1, wobei die elektrischen Wechselfelder für 3 bis 15 Tage angelegt werden, wobei die elektrischen Wechselfelder optional für 3 bis 10 Tage angelegt werden.

3. Checkpoint-Inhibitor zur Verwendung nach Anspruch 1 oder Anspruch 2, wobei die elektrischen Wechselfelder an jedem der Tage für mindestens 6 Stunden pro Tag an die Krebszellen angelegt werden.

4. Checkpoint-Inhibitor zur Verwendung nach Anspruch 1, wobei die elektrischen Wechselfelder für 3 Tage an die Krebszellen angelegt werden, gefolgt von einem Zeitraum von 3 Tagen, in dem die elektrischen Wechselfelder nicht an die Krebszellen angelegt werden, gefolgt von einem Zeitraum von 3 Tagen, in dem die elektrischen Wechselfelder an die Krebszellen angelegt werden.

5. Checkpoint-Inhibitor zur Verwendung nach Anspruch 1, wobei die elektrischen Wechselfelder mindestens 3 Tage pro Woche an die Krebszellen angelegt werden.

6. Checkpoint-Inhibitor zur Verwendung nach Anspruch 1, wobei die elektrischen Wechselfelder für einen ersten Zeitraum von mindestens 3 Tagen an die Krebszellen angelegt werden, gefolgt von einem zweiten Zeitraum, in dem die elektrischen Wechselfelder nicht angelegt werden.

7. Checkpoint-Inhibitor zur Verwendung nach Anspruch 6, wobei der zweite Zeitraum mindestens der gleiche ist wie der erste Zeitraum.

8. Checkpoint-Inhibitor zur Verwendung nach einem vorstehenden Anspruch, wobei die elektrischen Wechselfelder in kurzen Pulsen an die Krebszellen angelegt werden.

9. Checkpoint-Inhibitor zur Verwendung nach Anspruch 1, wobei die Krebszellen aus der Gruppe bestehend aus Glioblastomzellen, Pankreaskrebszellen, Eierstockkrebszellen, nicht-kleinzelligen Lungenkrebszellen (NSCLC) und Mesotheliomzellen ausgewählt sind.

10. Checkpoint-Inhibitor zur Verwendung nach Anspruch 1, wobei die Krebszellen Glioblastomzellen sind.

11. Checkpoint-Inhibitor zur Verwendung in einem Verfahren zum Behandeln von Glioblastomen, wobei das Verfahren Folgendes umfasst:
Anlegen von elektrischen Wechselfeldern an einen Kopf einer Zielperson mit Glioblastom bei einer Frequenz zwischen 100 und 500 kHz für mindestens 3 Tage; und
Verabreichen des Checkpoint-Inhibitors an die Zielperson, wobei das Verabreichen des Checkpoint-Inhibitors an die Zielperson nach Beendigung des Anlegens der elektrischen Wechselfelder erfolgt.

12. Checkpoint-Inhibitor zur Verwendung nach Anspruch 11, wobei die elektrischen Wechselfelder für 3 bis 15 Tage angelegt werden.

13. Checkpoint-Inhibitor zur Verwendung nach Anspruch 11, wobei die elektrischen Wechselfelder für 3 bis 10 Tage angelegt werden.

14. Checkpoint-Inhibitor zur Verwendung nach einem der Ansprüche 11 bis 13, wobei die elektrischen Wechselfelder kontinuierlich an die Zielperson angelegt werden.

15. Checkpoint-Inhibitor zur Verwendung nach einem der Ansprüche 11 bis 13, wobei die elektrischen Wechselfelder diskontinuierlich an die Zielperson angelegt werden.

16. Checkpoint-Inhibitor zur Verwendung nach Anspruch 15, wobei die elektrischen Wechselfelder an jedem der Tage für mindestens 4 Stunden pro Tag an die Zielperson angelegt werden.

17. Checkpoint-Inhibitor zur Verwendung nach einem der Ansprüche 11 bis 16, wobei der Checkpoint-Inhibitor aus der Gruppe bestehend aus Ipilimumab, Pembrolizumab und Nivolumab ausgewählt ist.

18. Checkpoint-Inhibitor zur Verwendung nach einem der Ansprüche 11 bis 17, wobei die elektrischen Wechselfelder eine Frequenz zwischen 180 und 220 kHz aufweisen.

19. Checkpoint-Inhibitor zur Verwendung nach Anspruch 11, wobei die elektrischen Wechselfelder für 3 Tage an den Kopf der Zielperson mit Glioblastom angelegt werden, gefolgt von einem Zeitraum von 3 Tagen, in dem die elektrischen Wechselfelder nicht an den Kopf der Zielperson mit Glioblastom angelegt werden, gefolgt von einem Zeitraum von 3 Tagen, in dem die elektrischen Wechselfelder an den Kopf der Zielperson mit Glioblastom angelegt werden.

20. Checkpoint-Inhibitor zur Verwendung nach Anspruch 11, wobei die elektrischen Wechselfelder in kurzen Pulsen an den Kopf der Zielperson mit Glioblastom angelegt werden.

21. Checkpoint-Inhibitor zur Verwendung nach Anspruch 11, wobei die elektrischen Wechselfelder mindestens 3 Tage pro Woche an den Kopf der Zielperson mit Glioblastom angelegt werden.

## Revendications

1. Inhibiteur de point de contrôle pour utilisation dans un procédé de traitement du cancer chez un sujet par réduction de la viabilité des cellules cancéreuses, le procédé comprenant :
l'application de champs électriques alternatifs aux cellules cancéreuses à une fréquence comprise entre 100 et 500 kHz pendant au moins 3 jours ; et l'administration de l'inhibiteur de point de contrôle au sujet,
dans lequel l'administration de l'inhibiteur de point de contrôle au sujet intervient après l'interruption de l'application des champs électriques alternatifs.

2. Inhibiteur de point de contrôle pour utilisation selon la revendication 1, dans lequel les champs électriques alternatifs sont appliqués pendant 3 à 15 jours, et éventuellement dans lequel les champs électriques alternatifs sont appliqués pendant 3 à 10 jours.

3. Inhibiteur de point de contrôle pour utilisation selon la revendication 1 ou la revendication 2, dans lequel les champs électriques alternatifs sont appliqués quotidiennement aux cellules cancéreuses pendant au moins 6 heures par jour.

4. Inhibiteur de point de contrôle pour utilisation selon la revendication 1, dans lequel les champs électriques alternatifs sont appliqués aux cellules cancéreuses pendant 3 jours, suivis d'une période de 3 jours où les champs électriques alternatifs ne sont pas appliqués aux cellules cancéreuses, suivie d'une période de 3 jours où les champs électriques alternatifs sont appliqués aux cellules cancéreuses.

5. Inhibiteur de point de contrôle pour utilisation selon la revendication 1, dans lequel les champs électriques alternatifs sont appliqués aux cellules cancéreuses au moins 3 jours par semaine.

6. Inhibiteur de point de contrôle pour utilisation selon la revendication 1, dans lequel les champs électriques alternatifs sont appliqués aux cellules cancéreuses pendant une première période d'au moins 3 jours, suivie d'une seconde période où les champs électriques alternatifs ne sont pas appliqués.

7. Inhibiteur de point de contrôle pour utilisation selon la revendication 6, dans lequel la seconde période est au moins aussi longue que la première période.

8. Inhibiteur de point de contrôle pour utilisation selon une quelconque revendication précédente, dans lequel les champs électriques alternatifs sont appliqués aux cellules cancéreuses par impulsions courtes.

9. Inhibiteur de point de contrôle pour utilisation selon la revendication 1, dans lequel les cellules cancéreuses sont sélectionnées dans le groupe consistant en des cellules de glioblastome, des cellules cancéreuses du pancréas, des cellules cancéreuses de l'ovaire, des cellules cancéreuses du poumon non à petites cellules (NSCLC) et des cellules de mésothéliome.

10. Inhibiteur de point de contrôle pour utilisation selon la revendication 1, dans lequel les cellules cancéreuses sont des cellules de glioblastome.

11. Inhibiteur de point de contrôle pour utilisation dans un procédé de traitement du glioblastome, le procédé comprenant :
l'application de champs électriques alternatifs à la tête d'un sujet atteint de glioblastome à une fréquence comprise entre 100 et 500 kHz pendant au moins 3 jours ; et
l'administration de l'inhibiteur de point de contrôle au sujet, dans lequel l'administration de l'inhibiteur de point de contrôle au sujet intervient après l'interruption de l'application des champs électriques alternatifs.

12. Inhibiteur de point de contrôle pour utilisation selon la revendication 11, dans lequel les champs électriques alternatifs sont appliqués pendant 3 à 15 jours.

13. Inhibiteur de point de contrôle pour utilisation selon la revendication 11, dans lequel les champs électriques alternatifs sont appliqués pendant 3 à 10 jours.

14. Inhibiteur de point de contrôle pour utilisation selon l'une quelconque des revendications 11 à 13, dans lequel les champs électriques alternatifs sont appliqués en continu au sujet.

15. Inhibiteur de point de contrôle pour utilisation selon l'une quelconque des revendications 11 à 13, dans lequel les champs électriques alternatifs sont appliqués au sujet de manière discontinue.

16. Inhibiteur de point de contrôle pour utilisation selon la revendication 15, dans lequel les champs électriques alternatifs sont appliqués quotidiennement au sujet pendant au moins 4 heures par jour.

17. Inhibiteur de point de contrôle pour utilisation selon l'une quelconque des revendications 11 à 16, dans lequel l'inhibiteur de point de contrôle est sélectionné dans le groupe consistant en l'ipilimumab, le pembrolizumab et le nivolumab.

18. Inhibiteur de point de contrôle pour utilisation selon l'une quelconque des revendications 11 à 17, dans lequel les champs électriques alternatifs présentent une fréquence comprise entre 180 et 220 kHz.

19. Inhibiteur de point de contrôle pour utilisation selon la revendication 11, dans lequel les champs électriques alternatifs sont appliqués à la tête du sujet atteint de glioblastome pendant 3 jours, suivis d'une période de 3 jours où les champs électriques alternatifs ne sont pas appliqués à la tête du sujet atteint de glioblastome, suivie d'une période de 3 jours où les champs électriques alternatifs sont appliqués à la tête du sujet atteint de glioblastome.

20. Inhibiteur de point de contrôle pour utilisation selon la revendication 11, dans lequel les champs électriques alternatifs sont appliqués à la tête du sujet atteint de glioblastome par impulsions courtes.

21. Inhibiteur de point de contrôle pour utilisation selon la revendication 11, dans lequel les champs électriques alternatifs sont appliqués à la tête du sujet atteint de glioblastome au moins 3 jours par semaine.
